# EUROPÄISCHE PATENTSCHRIFT

(11) **EP 2 871 231 B1**
(45) Veröffentlichungstag und Bekanntmachung des Hinweises auf die Patenterteilung: **13.02.2019**
(21) Anmeldenummer: 14192559.4
(22) Anmeldetag: 10.11.2014
(51) Int. Cl.: C12M 1/00, C12M 1/34, C12P 5/02, C12M 1/107

(54) **Verfahren zur Erzeugung von Biogas**
Method for generating biogas
Procédé de fabrication de biogaz

(30) Priorität: 08.11.2013 DE 102013018695
(43) Veröffentlichungstag der Anmeldung: 13.05.2015
(73) Patentinhaber: Senzyme GmbH, 53840 Troisdorf (DE)
(72) Erfinder: Lenz, Carl Jürgen, 53347 Alfter (DE); Hölker, Udo, 53639 Königswinter (DE); Janßen, Martina Iris, 53639 Königswinter (DE)
(74) Vertreter: Hauck Patentanwaltspartnerschaft mbB

(56) Entgegenhaltungen:
- EP-A1- 0 422 876
- WO-A1-2013/156784
- DE-A1-102004 042 688
- US-A1- 2013 029 314

## Beschreibung

Die Erfindung bezieht sich auf ein Verfahren zur Erzeugung von Biogas aus Biomasse in einem Biogasreaktor.

Der Prozess der Biogaserzeugung durch anaerobe Fermentation kann vereinfacht in vier Stufen unterteilt werden. In der ersten Stufe, der Hydrolyse, werden organische Makromoleküle (Polymere), aus welchen Biomasse besteht und welche in komplexer Art und Weise stofflich zusammengesetzt und untereinander vernetzt sind, in kleinere Bruchstücke (Oligomere und Dimere) und zuletzt in kleinste Einheiten (Monomere) zerlegt. Die wesentlichen Klassen dieser Makromoleküle sind Proteine, Lipide und Polysaccharide. Der Abbau der Makromoleküle erfolgt extrazellulär durch Enzyme, wobei diese teilweise in Form von Enzymkomplexen mit der Zellwand hydrolytisch aktiver Bakterien assoziiert, teilweise in gelöster Form vorliegen. Die durch die enzymatische Hydrolyse entstehenden, energiereichen Monomere wie Zucker, Zuckersäuren, Aminosäuren, Fettsäuren und andere organische Säuren sowie Alkohole werden in den weiteren Prozessschritten so umgewandelt, dass zuletzt Methan und Kohlendioxyd als wesentliche Bestandteile des gebildeten Biogases entstehen. Diese, der Hydrolyse nachgeordneten Prozessschritte werden als Acidogenese, Acetogenese und Methanogenese zusammengefasst und verlaufen intrazellulär.

Die technische Erzeugung von Biogas erfolgt in Biogasreaktoren. Dabei wird unter einem Biogasreaktor ein Behälter, vielfach individueller Bauart und Ausführung verstanden, der mit Inputstoffen betrieben und in dem der Biogasprozess ganz oder teilweise wie oben beschrieben durchgeführt wird. Unter Inputstoffen wird diejenige Biomasse pflanzlichen oder anderweitigen Ursprungs verstanden, die einem Biogasreaktor zugegeben und dort, in der Regel durch die Mitwirkung von Mikroorganismen, umgesetzt wird. Ein Biogasreaktor kann insbesondere ein Behälter zur Vorhydrolyse, ein Fermenter, ein Nachgärer oder ein Endlager sein. Es versteht sich, dass ein Biogasreaktor in eine Biogasanlage integriert ist, die insbesondere Einrichtungen zur Vorbehandlung der Inputstoffe, zur Einbringung der Inputstoffe in den Biogasreaktor, Rohrleitungen für den Transport von Biomasse und Biogas, Pumpen, Biogasreinigungssysteme und Blockheizkraftwerke umfassen kann.

Der Prozess der anaeroben Fermentation zum Zweck der Erzeugung von Biogas kann auf verschiedene Weise limitiert sein. Insbesondere kann die Hydrolyse limitierend auf den Gesamtprozess wirken. Bei der in der Praxis verbreiteten kontinuierlichen Betriebsweise von Biogasreaktoren zeigt sich eine Limitierung durch die Hydrolyse im Fließgleichgewicht zum Beispiel dadurch, dass sich flüchtige organische Fettsäuren nicht nachweisen lassen und zugleich die Biomasse in dem Biogasreaktor einen hohen Gehalt an organischer Trockensubstanz (im Vergleich zum Gehalt an organischer Trockensubstanz der Inputstoffe) und/oder eine hohe Zähigkeit aufweist. Eine hohe Zähigkeit der Biomasse in dem Bioreaktor erfordert eine hohe Rührenergie für die Homogenisierung der Biomasse, eine hohe Pumpenergie für die Förderung der Biomasse und fördert den Verschleiß von Pumpen und wirkt sich so hemmend auf den Gesamtprozess und damit auf dessen Wirtschaftlichkeit aus. Die mangelnde Bildung energiereicher Metabolite durch eine limitierende Hydrolyse schmälert die Effizienz der Substratverwertung und damit ebenfalls die Wirtschaftlichkeit des Gesamtprozesses. Im Nicht-Fließgleichgewicht zeigt sich eine Limitierung durch die Hydrolyse beispielsweise dadurch, dass sich Inhomogenitäten innerhalb des Gär-schlamms in einem Biogasreaktor ausbilden (Sink- oder Schwimm¬schichten aus nicht oder nur teilweise abgebauter Biomasse sowie Kegelbildungen an Feststoffeinträgen), der Biogasprozess also instabil ist. Bei der alternativen diskontinuierlichen Betriebsweise (Batch-Verfahren) zeigt sich eine Limitierung durch die Hydrolyse an der Zeitdauer, welche benötigt wird, bis eine erwartbare Menge Biogas mit einer erwartbaren Zusammensetzung aus den Inputstoffen gebildet worden ist, während zugleich die Konzentrationen gasförmiger (beispielsweise Wasserstoff) und gelöster Metabolite (beispielsweise flüchtige organische Fettsäuren), welche vorübergehend entstehen, niedrig sind (das Batch-Verfahren strebt einem thermodynamischen Gleichgewicht zu). Durch eine limitierende Hydrolyse wird unter anderem verhindert, das der Biogasprozess mit höheren Raumbelastungen betrieben, das heißt der Gesamtdurchsatz durch Steigerung der Fütterungsrate erhöht werden kann.

Es ist Stand der Technik, enzymhaltige Präparate dem Biogasprozess zu zugeben mit der Absicht, die Hydrolyse und dadurch den gesamten Biogasprozess zu verbessern. Extern zugegebene und sich in der wässrigen Phase der Biomasse in einem Biogasreaktor lösende Enzyme können in die molekulare, polymere Matrix der Biomasse hinein diffundieren und dort den Abbau der Makromoleküle beschleunigen, wohingegen an Zellwänden von Bakterien gebundene Enzymkomplexe nur dort wirken können, wo nicht aus sterischen Gründen ein Zugang zu den Substratmolekülen verhindert ist. Die primären Wirkungen hydrolytischer Enzyme auf die Biomasse sind die Spaltung von Makromolekülen und deren Quervernetzungen und die Freisetzung energiereicher Metabolite. Sekundäre Wirkungen sind zum Beispiel die Zerkleinerung makroskopischer und mikroskopischer Partikel, eine Verminderung der molekularen Scherkräfte innerhalb der Biomasse, Änderungen im Wasserbindeverhalten der Biomasse und des Bindevermögens für Ionen sowie eine Vergrößerung der effektiven Oberfläche der Biomasse.

Beispielsweise wird in WO2013000945 ein Verfahren beschrieben, bei dem ein oder mehrere Enzyme einem aus Bagasse hergestellten Schlamm im Zuge einer Vorbehandlung zugesetzt werden, mit der Absicht, den Biogasertrag aus der anaeroben Vergärung dieses Schlamms zu erhöhen. Das beschriebene Verfahren offenbart aber keine Methode, nach der die Enzymzugabe wirtschaftlich, d.h. unter Berücksichtigung von Kosten und Nutzen des Enzymeinsatzes, optimal gestaltet werden kann.

Unter dem Druck stetig steigender Agrarkosten, insbesondere der Rohstoffkosten, ist es jedoch notwendig, die Effizienz der Zugabe enzymhaltiger Präparate zu optimieren, das heißt, den Aufwand für eine Enzymzugabe zu minimieren und zugleich den wirtschaftlichen Nutzen zu maximieren.

Dies wird nach gängiger Praxis durch eine geeignete Zusammensetzung des oder der dem Biogasprozess zugegebenen enzymhaltigen Präparate angestrebt, das heißt durch die Auswahl geeigneter Enzymspektren der Präparate. Dabei orientieren sich die Enzymspektrum bisher ausschließlich an der Art und der stofflichen Zusammensetzung der Inputstoffe, der stofflichen Zusammensetzung der Biomasse in den Biogasreaktoren und der Art der Viskosität der Biomasse sowie der Art auftretender Inhomogenitäten innerhalb der Biomasse (Sink- oder Schwimmschichten).

Beispielsweise wird in DE 102004042688 ein Verfahren beschrieben, bei dem sich die spezifische Zusammensetzung eines enzymhaltigen Präparats zur Verbesserung der Homogenitätseigenschaften von Klärschlamm an der stofflichen Zusammensetzung des Klärschlamms, respektive den hydrolysierbaren Stofffraktionen, orientiert. Zwar wird hier eine Methode beschrieben, nach der besser geeignete Enzymprodukte ausgewählt werden können, es wird aber keine Methode offenbart, die es erlaubt, den wirtschaftlichen Einsatz insgesamt, also das Kosten/Nutzen-Verhältnis dadurch zu optimieren, dass die zugesetzte Menge des ausgewählten Enzymprodukts begrenzt wird.

Die US 2013/029314 A1 beschreibt eine Vorrichtung und ein Verfahren zur Biogaserzeugung. Die Anordnung und Steuerung von Rührern in einem Behälter (Fermenter) ist derart, dass die Durchmischung des Fermenterinhalts (Biomasse) und damit die Biogaserzeugung optimiert werden. Ferner ist beschrieben, dass Enzyme dem Fermenterinhalt im Rahmen der Biogaserzeugung zugesetzt werden können, um die Zusammensetzung des Fermenterinhalts zu verändern.
Die WO2013/156784 A1 beschreibt eine Vorrichtung und ein Verfahren zur anaeroben Behandlung wässriger, organischer Abfallströme u.a. mit dem Ziel der Biogaserzeugung. Die Vorrichtung ist ein horizontal angeordneter Behälter (Fermenter) mit mindestens einem Paddel zur Durchmischung und zum Vorschub des Fermenterinhalts sowie zur Erzeugung separater Reaktionszonen innerhalb des Behälters. Ferner enthält der Behälter Vorrichtungen zur Extraktion und Rezirkulation von Fermenterinhalt oder von erzeugtem Biogas. Parameter wie Temperatur, pH-Wert, Bakterien- und "Enzympopulationen" werden in den Reaktionszonen so kontrolliert, dass die dort stattfindenden Reaktionen optimal ablaufen. Dazu werden die Parameter gemessen und dazu verwendet, die Zugabe von Puffersubstanzen, Makro- und Mikroelementen, Bakterien und auch Enzymen zu steuern.

Hiervon ausgehend liegt der Erfindung die Aufgabe zugrunde, ein gegenüber dem Stand der Technik dadurch verbessertes Verfahren zur Erzeugung von Biogas in einem Biogasreaktor zur Verfügung zu stellen, dass es das Kosten/Nutzen-Verhältnis des Enzymeinsatzes optimiert.

Dies wird dadurch erreicht, dass Richtwerte für solche Merkmale theoretisch oder empirisch ermittelt und zur Verfügung gestellt werden, die die in kausalem Zusammenhang mit der Qualität der Hydrolyse stehen, dass für mindestens ein Merkmal der Merkmalswert in der Biomasse aus einem Biogasreaktor ermittelt wird und dass mindestens eine Methode angegeben wird, nach der eines oder mehrere enzymhaltige Präparate aus einer gegebenen Gruppe von mindestens zwei enzymhaltigen Präparaten so ausgewählt werden, dass bei Zugabe dieses oder dieser enzymhaltigen Präparate in den Biogasreaktor die für die Zurückführung einer etwaig gemessenen Abweichung des Merkmalswerts von dem zugehörigen Richtwert notwendige Gesamtmenge an enzymhaltigem Präparat oder enzymhaltigen Präparaten und/oder die Gesamtkosten minimal werden. Unter einem enzymhaltigen Präparat wird dabei vorzugsweise eine Zusammensetzung aus einem oder mehreren Enzymen mit oder ohne einen oder mehrere Trägerstoffe, Stabilisatoren, Konservierungsstoffe oder andere technische Hilfsstoffe in fester oder flüssiger Form verstanden. Das erfindungsgemäße Verfahren zur Erzeugung von Biogas in einem Biogasreaktor umfasst also die folgenden Schritte:
1) Mindestens ein Merkmal, bezogen auf die Biomasse aus einem Biogasreaktor, wird angegeben.
2) Mindestens ein Messverfahren für die Messung des mindestens einen Merkmals wird angegeben.
3) Mindestens ein Richtwert für das mindestens eine Merkmal wird zur Verfügung gestellt.
4) In dem Biogasreaktor wird Biogas erzeugt.
5) Der Wert des mindestens einen Merkmals in der Biomasse aus dem Biogasreaktor wird mit Hilfe des mindestens einen Messverfahrens ermittelt.
6) Mindestens ein Auswahlkriterium für die Wahl mindestens eines enzymhaltigen Präparates aus einer gegebenen Gruppe von mindestens zwei enzymhaltigen Präparaten wird zur Verfügung gestellt.
7) Falls der ermittelte Merkmalswert in der Biomasse von dem mindestens einen zur Verfügung gestellten Richtwert abweicht, werden das oder die gemäß des mindestens einen Auswahlkriteriums ausgewählten enzymhaltigen Präparate in einer solchen Menge oder über einen solchen Zeitraum der Biomasse in dem Biograsreaktor zugegeben, dass die gemessene Abweichung verschwindet.
8) Das Merkmal ist die Aktivität mindestens eines für die Hydrolyse maßgeblichen Enzyms oder einer maßgeblichen Gruppe von Enzymen in der Biomasse und dasjenige Enzym oder diejenige Enzymgruppe der Biomasse in dem Biogasreaktor wird zugegeben, für das oder für die die ermittelte Aktivität unterhalb des zur Verfügung gestellten Richtwertes liegt.

Überraschenderweise hat sich nämlich gezeigt, dass sich die Biogaserzeugung in einem bestimmten Biogasreaktor dann besonders effizient gestalten lässt, wenn Richtwerte für solche Merkmale eingehalten werden, die in direktem kausalen Zusammenhang mit der Qualität der Hydrolyse stehen (dadurch, dass sie Mitursache oder Folge der Qualität der Hydrolyse sind) und deren Abweichungen von den Richtwerten sich negativ auf die Hydrolyse und den Gesamtprozess auswirken und dass die Einstellung dieser Richtwerte durch die Zugabe eines oder mehrerer enzymhaltiger Präparate aus einer gegebenen Gruppe von Präparaten so erfolgt, dass die zugelegte Gesamtmenge des oder der enzymhaltigen Präparate und/oder die Gesamtkosten minimal sind. Dies wiederum wird dadurch erreicht, dass zu den Merkmalen jeweils passende Auswahlkriterien für die Auswahl des oder der Präparate zur Verfügung gestellt werden. Hierfür in Frage kommende Merkmale sind zum Beispiel die Aktivitäten für die Hydrolyse relevanter Enzyme, die Zähigkeit der Biomasse oder die spezifische Biogasbildung aus der Biomasse. Unter der Aktivität eines Enzyms wird hier ein Maß für die Umsatzgeschwindigkeit der durch dieses Enzym katalysierten biochemischen Reaktion verstanden, bezogen auf ein oder mehrere Substrate und gemessen mit einer geeigneten Methode. Unter dem Begriff Zähigkeit werden hier rheologische Eigenschaften, wie zum Beispiel die Viskosität, verstanden, welche mit einer geeigneten Methode in der Biomasse gemessen werden können. Unter spezifischer Biogasbildung wird beispielsweise die mit oder ohne Zugabe eines oder mehrerer Inputstoffe aus der Biomasse in einer bestimmten Zeit gebildete Menge an Methan verstanden, wie sie mit einer geeigneten Messapparatur ermittelt werden kann, bezogen auf einen Parameter der Biomasse (beispielsweise den Gehalt an organischer Trockenmasse). Unter einem Auswahlkriterium wird ein Verfahren verstanden, nach dem, bezogen auf das oder die gemessenen Merkmale, die Auswahl eines oder mehrerer enzymhaltigen Präparate so erfolgt, dass die für die Verminderung der Abweichung dieser Merkmalswerte von den zugehörigen Richtwerten notwendige Enzymmenge und/oder die Enzymkosten minimal sind. Zu den genannten Beispielen für Merkmale passende Auswahlkriterien beruhen im Falle der Wahl der Aktivitäten hydrolytischer Enzyme in der Biomasse aus einem Biogasreaktor als Merkmal beispielsweise auf der Abweichung der gemessenen Aktivitäten von den zur Verfügung gestellten Richtwerten, im Falle der Wahl der Zähigkeit der Biomasse aus einem Biogasreaktor als Merkmal beispielsweise auf der mittels einer geeigneten Apparatur gemessenen Abnahme der Zähigkeit in der Biomasse durch die Zugabe enzymhaltiger Präparate im Vergleich zu einer Kontrolle ohne Zugabe enzymhaltiger Präparate und im Falle der Wahl der spezifischen Biogasbildung aus der Biomasse aus einem Biogasreaktor als Merkmal beispielsweise auf der mit einer geeigneten Apparatur gemessenen spezifischen zusätzlichen Biogasbildung (Mehrbiogasbildung) durch die Zugabe enzymhaltiger Präparate im Vergleich zu einer Kontrolle ohne Zugabe enzymhaltiger Präparate.

Nach dem erfindungsgemäßen Verfahren werden die Werte eines oder mehrerer Merkmale in der Biomasse aus einem Biogasreaktor bestimmt. Dazu wird die Biomasse gegebenenfalls vorbehandelt, zum Beispiel bei einer bestimmten Temperatur gelagert, nass vermahlen, gesiebt, gemixt oder getrocknet oder es werden Stoffe, wie zum Beispiel Inputstoffe, zugesetzt und die Biomasse in Anwesenheit der Stoffe inkubiert. Bevorzugt werden die Inputstoffe oder anderen Stoffe vor der Zugabe vorbehandelt, zum Beispiel gemahlen. Bevorzugt werden die Inputstoffe zum Beispiel aus der Gruppe der folgenden Stoffe ausgewählt: Maissilage, Grassilage, Getreide-Ganzpflanzensilage, Grünroggen, Rindermist, Pferdemist, Stroh, Wegebegleitgrün, Grünabfall, Hühnertrockenkot, Rindergülle und Schweinegülle. Bevorzugt werden die anderen Stoffe zum Beispiel aus der Gruppe der folgenden Stoffe ausgewählt: Carboxymethylcellulose, mikrokristalline Cellulose, Xylan, Arabinoxylan, Pektin, Rhamnogalacturonan, Xyloglucan und Lignin. Weicht der gemessene Merkmalswert von dem zur Verfügung gestellten, zugehörigen Richtwert ab, so wird das gemäß des zugehörigen Auswahlkriteriums ausgewählte Enzym oder die ausgewählte Enzymgruppe dem Biogasreaktor solange zugegeben, bis der Merkmalswert den Richtwert erreicht. Dabei kann die Zugabe auf Fälle beschränkt werden, in denen eine deutliche Abweichung vom Richtwert (zum Beispiel um eine vorgegebene Toleranz) gegeben ist. Liegt die Abweichung vom Richtwert innerhalb des Toleranzbereiches, so kann von der Zugabe des Enzyms oder der Enzymklasse abgesehen werden. Bevorzugt wird für mehrere Enzyme oder Enzym-gruppen die Einhaltung der betreffenden Richtwerte kontrolliert und gegebenenfalls durch Zugabe von Enzymen oder Gruppen von Enzymen sichergestellt. Die Enzymzugabe dient der Verbesserung der Hydrolyse durch eine Erhöhung der Aktivitäten hydrolytisch aktiver Enzyme und/oder einer Verminderung der Zähigkeit der Biomasse und/oder einer Verbesserung der spezifischen Biogasbildung aus der Biomasse, und damit der Leistungssteigerung und Stabilisierung der Biogaserzeugung aus Biomasse in der Biogasanlage. Wird ein Enzymmangel in der Biomasse ausgeglichen, so wird die Hydrolyserate erhöht und der Gesamtdurchsatz in der Biogasanlage gesteigert. Dabei werden das oder diejenigen enzymhaltigen Präparate aus einer Gruppe vorhandener enzymhaltiger Präparate so ausgewählt und gegebenenfalls so kombiniert, dass für die Einstellung des Richtwertes rechnerisch die geringste Gesamtmenge an dem oder den enzymhaltigen Präparaten benötigt wird und/oder die Kosten minimal werden. Hierfür wird bevorzugt die Methode der Zielwertoptimierung angewendet.

Gemäß einer bevorzugten Ausführungsform des erfindungsgemäßen Verfahrens wird der Richtwert für ein Merkmal dadurch ermittelt, dass die Merkmalswerte für eine Stichprobe von Biomasseproben aus Biogasreaktoren verschiedener Biogasanlagen ermittelt werden, für die gemessenen Werte eine empirische Häufigkeitsverteilung erstellt wird und als Richtwert ein Quantil der empirischen Häufigkeitsverteilung gewählt wird. Dabei sollte der Stichprobenumfang so groß sein, dass der höchste gemessene Wert des Merkmals mindestens das 10-fache des kleinsten gemessenen Wertes beträgt.

Es ist anzunehmen, dass durch weitere Untersuchungen weitere Erkenntnisse gewonnen werden können, welche die Bereitstellung weiterer geeigneter Merkmale, Messmethoden, genauere Richtwerte und bessere Auswahlkriterien zur Auswahl der enzymhaltigen Präparate ermöglichen.

### Ausgestaltung I: Merkmal ist die Aktivität mindestens eines für die Hydrolyse relevanten Enzyms oder einer relevanten Enzymgruppe:

Gemäß einer bevorzugten Ausgestaltung I des erfindungsgemäßen Verfahrens ist das mindestens eine messbare Merkmal die Aktivität mindestens eines für die Hydrolyse relevanten Enzyms oder einer relevanten Gruppe von Enzymen in einer Biomasseprobe aus einem Biogasreaktor. Die Ausgestaltung I des erfindungsgemäßen Verfahrens geht von der überraschenden Erkenntnis aus, dass die Biogaserzeugung in einem Biogasreaktor dann besonders effektiv ist, wenn die Aktivität mindestens eines für die Hydrolyse relevanten Enzyms oder einer relevanten Gruppe von Enzymen einen empirisch oder theoretisch ermittelten oder definierten Richtwert einhält. Dabei werden die Aktivitäten der Enzyme oder Enzymgruppen in der Biomasse aus einem Biogasreaktor mittels eines geeigneten Verfahrens bestimmt, beispielsweise durch die Zugabe des zugehörigen Substrats (beispielsweise Carboxymethylcellulose zur Messung der Cellulaseaktivität, Birkenxylan zur Messung der Xylanaseaktivität, Apfelpektin zur Messung der Pektinaseaktivität) und die photometrische Messung der Reaktionsgeschwindigkeit. Bevorzugt wird die Biomasse aus dem Biogasreaktor zuvor zentrifugiert und die Enzymaktivitäten im Überstand nach der Zentrifugation bestimmt, weil die lösliche Enzymfraktion die sich im Überstand befindet diejenige ist, die durch die Zugabe von Enzymen in die Biomasse eines Biogasreaktors beeinflusst wird.

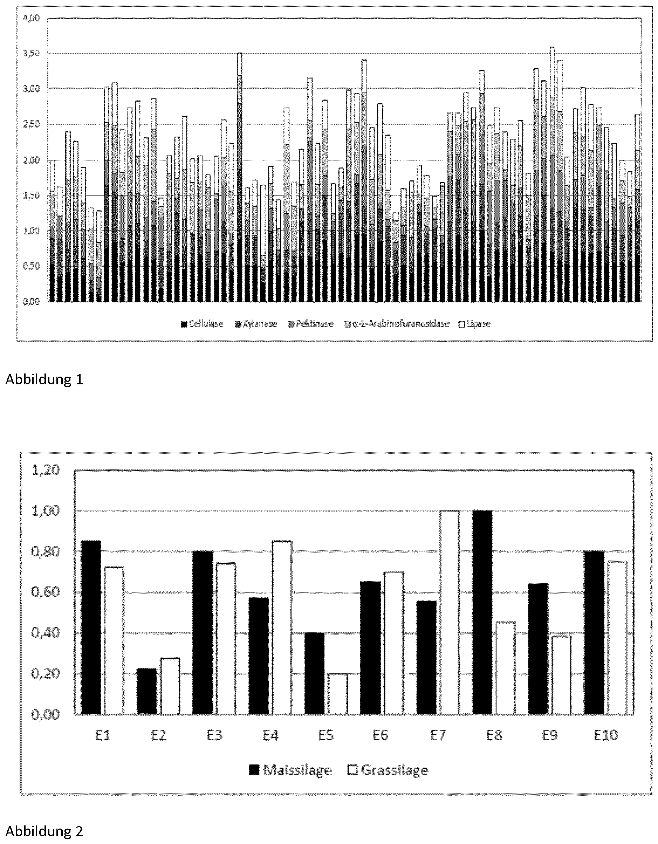

Die Aktivitäten von für die Hydrolyse relevanten Enzymen bzw. Gruppen von Enzymen wurden im Rahmen einer Untersuchung an Biogasreaktoren von 76 Praxisanlagen ermittelt. Überraschenderweise hat diese Untersuchung gezeigt, dass die Aktivitäten löslicher hydrolytischer Enzyme bzw. Gruppen von Enzymen in Proben aus den Biogasreaktoren erheblichen Schwankungen unterworfen sind. Dies zeigt die Abbildung 1 in Anhang 1 für die Enzyme bzw. Enzymgruppen Cellulase, Xylanase, Pektinase, α-L-Arabinofuranosidase und Lipase. Hierbei sind die Enzyme bzw. Enzymgruppen Cellulase, Xylanase, Pektinase und α-L-Arabinofuranosidase funktionell am Abbau der pflanzlichen Zellwand beteiligt, während die Lipase am Fettabbau beteiligt ist. Die in den untersuchten Biogasreaktoren gemessenen Enzymaktivitäten bzw. Aktivitäten von Enzymgruppen variieren um mehr als eine Größenordnung, wobei es vorkommt, dass eine Probe aus einem Biogasreaktor bezüglich eines Enzyms bzw. einer Enzymgruppe eine hohe Aktivität, gemessen an der höchsten in der Stichprobe gemessenen Aktivität dieses Enzyms oder dieser Enzymgruppe, besitzt, während dieselbe Probe bezüglich eines anderen Enzyms bzw. Enzymgruppe eine niedrige Aktivität besitzt. Ebenso überraschend hat sich im Rahmen der Untersuchung gezeigt, dass ein Zusammenhang zwischen den Aktivitäten der untersuchten Enzyme bzw. Enzymgruppen in den Biomasseproben aus den Biogasreaktoren und der relativen Substratausnutzung der zu den Proben gehörenden Biogasanlagen besteht. Dabei ist die relative Substratausnutzung ein Maß für die Leistung einer Biogasanlage und zum Beispiel definierbar als diejenige Menge an Methan, welche aus den in einer Biogasanlage eingesetzten Inputstoffen relativ zu der erwartbaren Menge an Methan pro Zeiteinheit gebildet wird. Die für jeden Inputstoff erwartbare Menge an Methan lässt sich zum Beispiel dem KTBL-Heft 50, Gasausbeute in landwirtschaftlichen Biogasanlagen, Herausgeber Kuratorium für Technik und Bauwesen in der Landwirtschaft e. V., Darmstadt, 2005 entnehmen. Der Mittelwert der Verteilung der relativen Substratausnutzung wurde durch Hölker (2013, persönliche Mitteilung) in einer umfangreichen Reihenuntersuchung ermittelt und liegt für die untersuchte Stichprobe von etwa 2.000 landwirtschaftlichen Biogasanlagen in Deutschland etwa bei 109%. Für die hier vorliegende Untersuchung wurde vereinfachend relativen Substratausnutzungen über 109% der Rang "gut" und relativen Substratausnutzungen unter 109% der Rang "schlecht" zugeordnet. Dann wurde die Ausprägung des Rangs innerhalb der untersuchten Biogasanlagen mit den in den Proben aus den zugehörigen Biogasreaktoren gemessenen Enzymaktivitäten bzw. Aktivitäten der Enzymgruppen dadurch in Beziehung gesetzt, dass die Korrelationskoeffizienten nach Pearson zwischen dem Rang und der jeweiligen Enzymaktivität bzw. Aktivität der Enzymgruppe ermittelt wurden. Der Wert des Korrelationskoeffizienten nach Pearson liegt zwischen -1 und 1, wobei der Wert -1 eine maximal negative Korrelation und der Wert +1 eine maximal positive Korrelation bedeuten und der Wert 0 bedeutet, dass die Merkmale nicht miteinander korrelieren, das heißt in keinem kausalen Zusammenhang miteinander stehen. Es wurden folgende Korrelationskoeffizienten zwischen den gemessenen Enzymaktivitäten und der relativen Substratausnutzung gemessen: Cellulase 0,20; Xylanase 0,13; Pektinase 0,13; α-L-Arabinofuranosidase 0,15; Lipase 0,02. Dabei zeigt sich, dass zwischen den gemessenen Enzymaktivitäten von Cellulase, Xylanase, Pektinase und α-L-Arabinofuranosidase und der relativen Substratausnutzung eine positive Korrelation besteht, während die Aktivität der Lipase nicht mit der relativen Substratausnutzung korreliert. Daraus lässt sich schließen, dass die Aktivitäten zellwandabbauender Enzyme bzw. Enzymgruppen einen wesentlichen, kausalen Faktor für die Gesamtleistung einer Biogasanlage darstellen und dass niedrige Aktivitäten zellwandabbauender Enzyme in dieser Hinsicht, das heißt bezogen auf die Anlagenleistung, als ein Defizit zu deuten sind. Dieser Befund wird unterstützt durch die fehlende Korrelation zwischen der Lipaseaktivität und der relativen Substratausnutzung, denn der Fettabbau, der durch die Lipase katalysiert wird, ist beim Einsatz pflanzlicher Inputstoffe in landwirtschaftlichen Biogasanlagen in der Regel nicht limitierend. Bei den 76 untersuchten Biogasanlagen handelte es sich um Biogasanlagen, welche pflanzliche Inputstoffe, hauptsächlich Maissilage und Grassilage, einsetzen. Aus diesen Ergebnissen folgt, dass sich Richtwerte für die Aktivität mindestens eines für die Hydrolyse relevanten Enzyms oder einer relevanten Enzymgruppe theoretisch oder empirisch ableiten oder definieren lassen, deren Einhaltung das Verhältnis aus Leistung einer Biogasanlage und wirtschaftlichem Aufwand für den Enzymeinsatz in der Praxis optimiert.

Gemäß einer bevorzugten Ausführungsform der Ausgestaltung I des erfindungsgemäßen Verfahrens ist der Richtwert für die Aktivität des mindestens einen Enzyms oder der mindestens einen Gruppe von Enzymen in der Biomasse das 75%-Quantil der empirischen Häufigkeitsverteilung der Aktivität innerhalb einer Stichprobe von Biogasanlagen. Ist die in der Biomasseprobe aus einem Biogasreaktor gemessene Aktivität des mindestens einen Enzymes oder der mindestens einer Enzymgruppe kleiner als der zugehörige Richtwert, werden ein oder mehrere enzymhaltige Präparate, welche dieses Enzym oder diese Gruppe von Enzymen enthalten, dem Biogasreaktor in einer solchen Menge oder über einen solchen Zeitraum zugegeben, dass die gemessene Abweichung verschwindet. Dabei werden das oder die enzymhaltigen Präparate so aus der Gruppe der zur Verfügung stehenden enzymhaltigen Präparate ausgewählt, dass die für die Rückführung der Abweichung notwendige Gesamtmenge und/oder die Gesamtkosten minimal werden. Bevorzugt wird deshalb für die Auswahl des oder der enzymhaltigen Präparate die Methode der Zielwertoptimierung angewendet. Beispiel 1 erläutert die hier beschriebene bevorzugte Ausführungsform der Ausgestaltung I, ohne dass die Verfahrensausgestaltung auf dieses Beispiel beschränkt wäre.

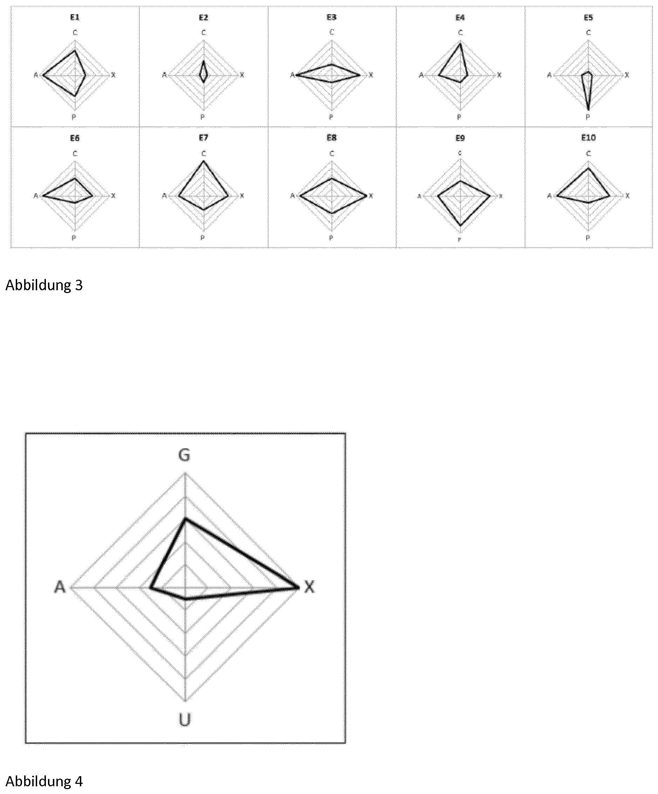

Gemäß einer anderen Ausführungsform der Ausgestaltung I des erfindungsgemäßen Verfahrens werden die Richtwerte empirisch aus der Wirkung enzymhaltiger Präparate auf den Abbau bestimmter, in Biogasanlagen eingesetzter Inputstoffe (beispielsweise Maissilage oder Grassilage) oder Inputstoffmischungen abgeleitet. Auf diese Weise erhält man inputstoffspezifische Richtwerte. Im Rahmen eines Versuchsprogramms wurde beispielsweise die Wirkung verschiedener enzymhaltiger Präparate auf die Zerkleinerung der Inputstoffe Maissilage und Grassilage untersucht. Dazu wurde Maissilage bzw. Grassilage mit den verschiedenen enzymhaltigen Präparaten 24 h bei 40°C unter Rühren inkubiert und im Anschluss nach Zentrifugieren des Ansätze in den Überständen die durch die Enzymwirkung aus dem Abbau von Cellulose, Glucan, Xylan und Pektin freigesetzten Mengen an reduzierenden Zuckern (Verzuckerungsgrad) mit Hilfe von Di-Nitro-Salicylsäure in einem photometrischen Nachweisverfahren bestimmt. Wie Abbildung 2 in Anhang I zeigt, unterscheiden sich die Verzuckerungsgrade je nach eingesetztem enzymhaltigen Präparat erheblich. Auch unterscheiden sich die Verzuckerungsgrade abhängig vom eingesetzten Inputstoff. Diese unterschiedliche Wirkung der verschiedenen enzymhaltigen Präparate auf den Verzuckerungsgrad von Maissilage bzw. Grassilage beruht auf ihrem Enzymprofil, das heißt ihrem Gehalt an verschiedenen zellwandabbauenden Enzymen oder Enzymgruppen. Die Enzymprofile der eingesetzten enzymhaltigen Präparate sind in Abbildung 3 in Anhang I am Beispiel der Enzyme bzw. Enzymgruppen Cellulase, Xylanase, Pektinase, α-L-Arabinofuranosidase gezeigt. Dabei sind die Enzymaktivitäten in relativen Einheiten in Form einer Netzgrafik dargestellt, wobei die Lage eines Punktes auf einer Ecke der Netzgrafik bedeutet, dass die entsprechende Aktivität des Enzyms oder der Enzymgruppe innerhalb der Gruppe der eingesetzten enzymhaltigen Präparate den höchsten Wert hatte. In der Netzgrafik entspricht dies dem Wert 1. Aus Abbildung 2 geht hervor, dass das enzymhaltige Präparat E7 den höchsten Verzuckerungsgrad an Grassilage erzielte, während das enzymhaltige Präparat E8 den höchsten Verzuckerungsgrad an Maissilage erzielte. Die Enzymprofile der Präparate E7 und E8 können deshalb als Zielenzymprofile für den Abbau von Grassilage bzw. Maissilage in Biogasanlagen aufgefasst werden. Gemäß der hier beschriebenen Ausführungsform der Ausgestaltung I des erfindungsgemäßen Verfahrens werden die Richtwerte für zellwandabbauende Enzyme oder Enzymgruppen aus den Zielenzymprofilen für den Abbau von Inputstoffen abgeleitet und bevorzugt dann angewendet, wenn im konkreten Fall eine Biogasanlage den entsprechenden Inputstoff (beispielsweise Maissilage oder Grassilage) überwiegend, das heißt zum Beispiel mit einem Trockensubstanzanteil von 80% an der Gesamttrockenmasse aller eingesetzter Inputstoffe einsetzt. Beispiel 2 zeigt beispielhaft, wie sich die Richtwerte aus den Zielenzymprofilen ableiten lassen, ohne dass die Verfahrensausgestaltung auf hierauf beschränkt wäre. Für den Fall, dass in einer Biogasanlage eine Mischung von Inputstoffen eingesetzt wird (Inputstoffspektrum), ohne dass ein Inputstoff dominiert, können Zielenzymprofile für das Inputstoffspektrum dadurch erzeugt werden, dass die Enzymprofile für die einzelnen Inputstoffe im Verhältnis der jeweiligen Mengenanteile der Inputstoffe linear kombiniert werden. Auch für diesen Fall ist in Beispiel 2 eine Methode beispielhaft ausgeführt, ohne dass die Verfahrensausgestaltung auf hierauf beschränkt wäre.

Gemäß einer weiteren Ausführungsform der Ausgestaltung I des erfindungsgemäßen Verfahrens werden die Richtwerte empirisch aus der stofflichen Zusammensetzung der Inputstoffe oder der Biomasse in einem Biogasreaktor abgeleitet. Auf diese Weise erhält man stoffspezifische Richtwerte. Bevorzugt hierfür werden hierfür beispielsweise die Gehalte an Zuckern und Zuckersäuren bestimmt, wie zum Beispiel Glucose, Xylan, Galacturonsäure und Arabinose. So erhält man ein Stoffprofil der Inputstoffe oder der Biomasse in einem Biogasreaktor, wie es zum Beispiel in Abbildung 4 gezeigt ist. Alternativ können auch die Gehalte an Polymeren wie beispielsweise Cellulose, Glucan, Xylan, Arabinoxylan und Pektin bestimmt werden. Gemäß der hier beschriebenen Ausführungsform der Ausgestaltung I des erfindungsgemäßen Verfahrens werden die Richtwerte für zellwandabbauende Enzyme oder Enzymgruppen aus dem Stoffprofil der Inputstoffe oder der Biomasse in einem Biogasreaktor abgeleitet. Beispiel 3 zeigt beispielhaft, wie sich die Richtwerte aus dem Stoffprofil ableiten lassen, ohne dass die Verfahrensausgestaltung hierauf beschränkt wäre.

### Ausgestaltung II: Merkmal ist mindestens eine rheologische Eigenschaften der Biomasse, gemessen in einer geeigneten Apparatur:

Gemäß einer weiteren bevorzugten Ausgestaltung II des erfindungsgemäßen Verfahrens ist das mindestens eine messbare Merkmal eine rheologische Eigenschaft der Biomasse aus einem Biogasreaktor, gemessen in einer geeigneten Apparatur, beispielsweise mit Hilfe eines Viskosimeters, wie eines Rotationsviskosimeters, eines Drehmomentviskosimeters, eines Auslauftrichters, eines Setzfließmaßes oder einer schrägen Rampe. Die Ausgestaltung II des erfindungsgemäßen Verfahrens geht dabei von der überraschenden Erkenntnis aus, dass die Biogaserzeugung in einem Biogasreaktor dann besonders effektiv ist, wenn mindestens eine rheologische Eigenschaft der Biomasse einen empirisch oder theoretisch ermittelten Richtwert einhält. Bevorzugt wird die Viskosität in der Biomasse mittels eines Rotationsviskosimeters, nach vorhergehender Zerkleinerung der Biomasse mittels eines Thermomixers, gemessen. Bevorzugt wird die Zähigkeit bei 40°C bestimmt.

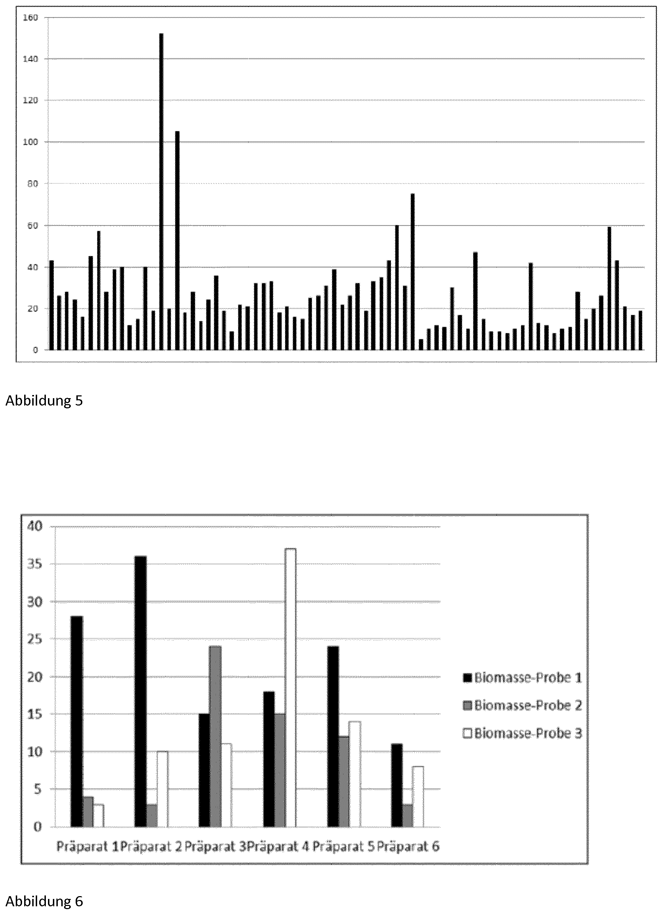

Die Viskosität der Biomasse wurde im Rahmen einer Untersuchung an Biogasreaktoren von 76 Praxisanlagen mit Hilfe eines Rotationsviskosimeters nach vorhergehender Zerkleinerung mittels eines Thermomixers bei 40°C ermittelt. Diese Untersuchung ergab, dass die Viskosität erheblichen Schwankungen unterworfen ist. Dies zeigt die Abbildung 5 in Anhang 1. Überraschenderweise hat sich im Rahmen der Untersuchung auch gezeigt, dass ein Zusammenhang zwischen den Viskosität der Biomasseproben aus den Biogasreaktoren und der relativen Substratausnutzung der zu den Proben gehörenden Biogasanlagen besteht (Korrelationskoeffizient nach Pearson -0,32). Dies zeigt, dass die Viskosität in einem Biogasreaktor einen wesentlichen, kausalen Faktor für die Gesamtleistung einer Biogasanlage darstellt und nicht nur technische Parameter, wie den Energieverbrauch für die Rührerleistung, die Pumpenergie für die Biomasse und den Verschleiß von Pumpen sondern auch die biologische Abbaurate der Inputstoffe in einem Biogasreaktor beeinflusst. Und zwar sinkt die Abbaurate mit zunehmender Viskosität, sodass sich die relative Substratausnutzung verschlechtert. Aus diesem Ergebnis folgt, dass sich Richtwerte für die Viskosität theoretisch oder empirisch ableiten oder definieren lassen, deren Einhaltung durch eine geeignete Zugabe enzymhaltiger Präparate die wirtschaftliche Leistung einer Biogasanlage in der Praxis optimiert.

Gemäß der bevorzugten Ausführungsform der Ausgestaltung II des erfindungsgemäßen Verfahrens ist der Richtwert für die Viskosität in der Biomasse das 45%-Quantil der empirischen Häufigkeitsverteilung der Viskosität innerhalb einer Stichprobe von Biogasanlagen. Bevorzugt die Auswahl des oder der Enzympräparate so vorgenommen, dass aus einer Gruppe von mindestens zwei enzymhaltigen Präparaten mindestens zwei Präparate einzeln und/oder in Kombination, wobei die Kombinationen unterschiedliche Anteile der mindestens zwei enzymhaltigen Präparate enthalten können, der Biomasse aus dem Biogasreaktor in einer geeigneten Apparatur zugesetzt werden und Änderung der Zähigkeit gegenüber einer Kontrolle ohne Zugabe eines enzymhaltigen Präparats nach einer Inkubationszeit ermittelt wird. Bevorzugt wird die Viskosität mittels eines Rotationsviskosimeters nach Zerkleinerung der Biomasseprobe mittels eines Thermomixers bestimmt. Bevorzugt liegt die Inkubationszeit zwischen 24 und 140 Stunden. Bevorzugt liegt die Inkubationstemperatur bei 40°C. Es wird dasjenige enzymhaltige Präparat oder die Kombination enzymhaltiger Präparate aus der Gruppe der untersuchten enzymhaltiger Präparate der Biomasse in dem Biogasreaktor zugesetzt, welches oder welche die stärkste Absenkung der Viskosität nach der Inkubation gegenüber der Kontrolle ergibt. Überraschenderweise hat sich nämlich gezeigt, dass sich die Wirkung verschiedener enzymhaltiger Präparate auf die Absenkung der Viskosität in einer Biomasseprobe, gemessen mittels eines Rotationsviskosimeters, im Vergleich zur Kontrolle ohne Zusatz des enzymhaltigen Präparats für Biomasseproben aus verschiedenen Biogasreaktoren erheblich unterscheiden kann. Dies zeigt die Abbildung 6 in Anhang 1. Beispiel 4 erläutert die hier beschriebene Ausführungsform der Ausgestaltung II, ohne dass die Verfahrensausgestaltung auf dieses Beispiel beschränkt wäre.

### Ausgestaltung III: Merkmal ist die spezifische Bildung von Biogas aus der Biomasse aus einem Biogasreaktor, gemessen in einer geeigneten Apparatur:

Gemäß einer weiteren bevorzugten Ausgestaltung III des erfindungsgemäßen Verfahrens ist das mindestens eine messbare Merkmal die spezifische Biogasbildung aus der Biomasse aus einem Biogasreaktor, gemessen in einer geeigneten Apparatur nach einer Inkubationszeit, beispielsweise mit Hilfe des ANKOM-Systems (für eine Beschreibung siehe zum Beispiel unter www.ankom.com) oder eines Eudiometers. Ausgestaltung III geht dabei von der überraschenden Erkenntnis aus, dass die Biogaserzeugung in einem Biogasreaktor dann besonders effektiv ist, wenn die spezifische Biogasbildung aus der Biomasse einen empirisch oder theoretisch ermittelten Richtwert einhält. Bevorzugt wird die spezifische Biogasbildung mit Hilfe des ANKOM-Systems nach einer Inkubationszeit von 24-140 Stunden bei 40°C gemessen. Der Biomasse können auch Stoffe zugegeben werden, beispielsweise Inputstoffe wie Maissilage oder Grassilage oder Stoffe wie Carboxymethylcellulose, mikrokristalline Cellulose, Xylan, Arabinoxylan oder Pektin.

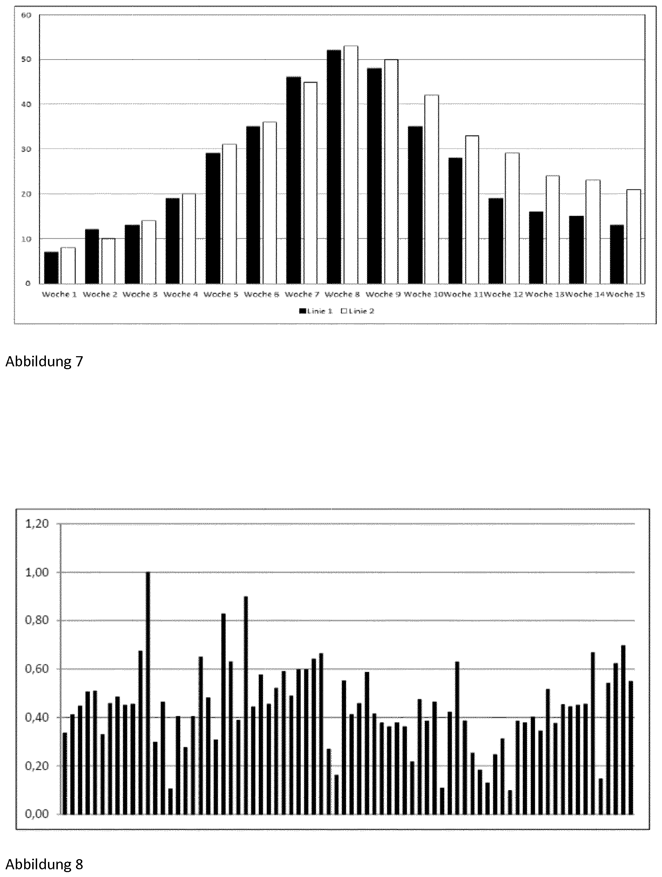

Die spezifische Biogasbildung wurde im Rahmen einer Untersuchung von Biomasseproben aus Biogasreaktoren von 76 Praxisanlagen mit Hilfe des ANKOM-Systems nach 140 Stunden Inkubation bei 40°C gemessen. Dabei zeigte sich, dass die spezifische Biogasbildung erheblichen Schwankungen unterworfen ist. Dies zeigt die Abbildung 8 in Anhang 1. Überraschenderweise hat sich im Rahmen der Untersuchung auch gezeigt, dass ein Zusammenhang zwischen der spezifischen Biogasbildung und der relativen Substratausnutzung der zu den Proben gehörenden Biogasanlagen besteht (Korrelationskoeffizient nach Pearson 0,25). Dies zeigt, dass die spezifische in einem Biogasreaktor Maß für die Gesamtleistung einer Biogasanlage darstellt. Eine hohe spezifische Biogasbildung innerhalb kurzer Inkubationszeiten deutet auf eine hohe Hydrolyserate hin, solange garantiert ist, dass dabei entstehende, energiereiche Metabolite sich nicht anhäufen. Aus diesem Ergebnis folgt, dass sich Richtwerte für die spezifische Biogasbildung theoretisch oder empirisch ableiten lassen, deren Einhaltung durch eine geeignete Zugabe enzymhaltiger Präparate die wirtschaftliche Leistung einer Biogasanlage in der Praxis optimiert.

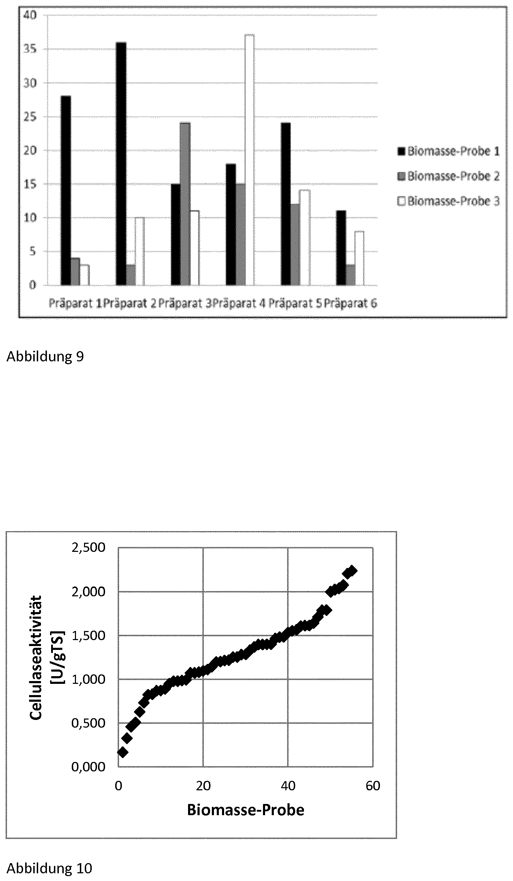

Gemäß der bevorzugten Ausführungsform der Ausgestaltung III des erfindungsgemäßen Verfahrens ist der Richtwert für die spezifische Biogasbildung aus der Biomasse das 75%-Quantil der empirischen Häufigkeitsverteilung der spezifischen Biogasbildung innerhalb einer Stichprobe von Biogasanlagen. Bevorzugt die Auswahl des oder der enzymhaltigen Präparate so vorgenommen, dass aus einer Gruppe von mindestens zwei enzymhaltigen Präparaten mindestens zwei Präparate einzeln und/oder in Kombination, wobei die Kombinationen unterschiedliche Anteile der mindestens zwei enzymhaltigen Präparate enthalten können, der Biomasse aus dem Biogasreaktor in einer geeigneten Apparatur zugesetzt werden und spezifische Mehrbiogasbildung gegenüber einer Kontrolle ohne Zugabe eines enzymhaltigen Präparats nach einer Inkubationszeit ermittelt wird. Bevorzugt wird die spezifische Mehrbiogasbildung mittels des ANKOM-Systems bestimmt. Bevorzugt liegt die Inkubationszeit zwischen 24 und 140 Stunden. Bevorzugt liegt die Inkubationstemperatur bei 40°C. Es wird dasjenige enzymhaltige Präparat oder die Kombination enzymhaltiger Präparate aus der Gruppe der untersuchten enzymhaltiger Präparate der Biomasse in dem Biogasreaktor zugesetzt, welches oder welche die höchste spezifische Mehrbiogasbildung nach der Inkubation gegenüber der Kontrolle ergibt. Überraschenderweise hat sich nämlich gezeigt, dass sich die Wirkung verschiedener enzymhaltiger Präparate auf die Mehrbiogasbildung aus einer Biomasseprobe, gemessen mittels des ANKOM-Systems, im Vergleich zur Kontrolle ohne Zusatz des enzymhaltigen Präparats für Biomasseproben aus verschiedenen Biogasreaktoren erheblich unterscheiden kann. Dies zeigt die Abbildung 9 in Anhang 1. Beispiel 5 erläutert die hier beschriebene Ausführungsform der Ausgestaltung III, ohne dass die Verfahrensausgestaltung auf dieses Beispiel beschränkt wäre.

### Weitere Ausgestaltungen, welche sich auf das Verfahren insgesamt, unabhängig von den Ausgestaltungen I-III, beziehen:

Gemäß einer bevorzugten Ausgestaltung des erfindungsgemäßen Verfahrens werden die Häufigkeitsverteilungen der Merkmale und die Richtwerte für verschiedene Gruppen von Biogasanlagen aus einer Stichprobe getrennt ermittelt, wobei eine Gruppe von Biogasanlagen dadurch definiert ist, dass diese Biogasanlagen mindestens eine Eigenschaft gemeinsam haben. Bevorzugt wird die mindestens eine Eigenschaft zum Beispiel aus der Gruppe folgender Eigenschaften ausgewählt: Art, Menge und Qualität der Inputstoffe, Art der Vorbehandlung der Inputstoffe, Anzahl hintereinander geschalteter Biogasreaktoren, Raumbelastung des Fermenters, Verweilzeiten in den Biogasreaktoren, Art der verwendeten Rührwerke, Betriebstemperatur in den Biogasreaktoren, Einsatz prozessfördernder Präparate, stoffliche und biochemische Parameter des Gärschlamms (zum Beispiel Gehalt an Trockensubstanz, Gehalt an organischer Trockensubstanz, Rohfasergehalt, NDF, ADF, ADL, Gehalt an Hemicellulosen, Gehalt an Lignin, pH-Wert, elektrische Leitfähigkeit, Ammoniumgehalt, Gehalt an anorganischem Puffer, Gehalt an Makroelementen, Gehalt an Spurenelementen, Gehalt an Metaboliten) und physikalische Parameter des Gärschlamm (rheologische Parameter, Partikelgrößenverteilung, Sedimentationsgrad, Entwässerungsvermögen).

Gemäß einer anderen bevorzugten Ausgestaltung des erfindungsgemäßen Verfahrens werden die in einer Stichprobe von Biomasseproben aus Biogasreaktoren verschiedener Biogasanlagen gemessenen Werte für ein oder mehrere Merkmale mit den Aktivitäten von für die Hydrolyse relevanten Enzymen oder Gruppen von Enzymen korreliert, beispielsweise, in dem der Korrelationskoeffizient nach Pearson oder der lineare Regressionskoeffizient ermittelt werden. Auf Basis dieser ermittelten Korrelationskoeffizienten oder linearen Regressionskoeffizienten werden dasjenige Enzym oder die Enzymgruppe für die Zugabe in die Biomasse eines Bioreaktors ausgewählt, für welche der ermittelte Korrelationskoeffizient oder lineare Regressionskoeffizient oberhalb eines Schwellenwertes liegt. Beispielsweise wird der Biomasse aus dem Biogasreaktor in einer geeigneten Apparatur wie beschrieben Inputstoff zugesetzt, die Biogasbildung nach einer vorgegebenen Inkubationszeit gemessen und für die zu jedem Inputstoff gehörende Messreihe die Korrelationskoeffizienten (nach Pearson) oder die linearen Regressionskoeffizienten der spezifischen Biogaserträge mit den in der Biomasse ermittelten Aktivitäten der Enzyme oder Enzymgruppen bestimmt. Auf Basis der ermittelten Korrelationskoeffizienten oder linearen Regressionskoeffizienten werden dasjenige Enzym oder die Enzymgruppe für die Zugabe in die Biomasse eines bestimmten Bioreaktors ausgewählt, für welche a) der ermittelte Korrelationskoeffizient oder lineare Regressionskoeffizient oberhalb eines Schwellenwertes liegt und b) die betreffende Biogasanlage, aus der die Biomasse entnommen wurde, denjenigen Inputstoff in einer relevanten Menge einsetzt, für den ein Korrelationskoeffizient oder linearer Regressionskoeffizient oberhalb des jeweiligen Schwellenwertes ermittelt wurde. "Relevant" bedeutet hierbei, dass die Biogasanlage den Inputstoff in einem Anteil von bevorzugt mindestens 20% der Trockenmasse. Bevorzugt werden Maissilage oder Grassilage als die am häufigsten in der Praxis in landwirtschaftlichen Biogasanlagen eingesetzten Inputstoffe für diese Ausgestaltung verwendet und der Biogasertrag mit Hilfe des ANKOM-Verfahrens ermittelt.

Gemäß einer weiteren bevorzugten Ausgestaltung des erfindungsgemäßen Verfahrens werden die ermittelten Richtwert nachträglich mit Faktoren multipliziert. Beispielsweise werden können die Richtwerte mit einem Faktor multipliziert werden, der von der Raumbelastung einer Biogasanlage abhängt. Die Raumbelastung gibt die pro Behältervolumen eines Bioreaktors täglich zugeführte Menge an Trockenmasse an. Die Richtwerte können auch mit Faktoren multipliziert werden, die von der hydraulischen Verweilzeit oder dem Trockenmassegehalt in einem Biogasreaktor abhängen. Die Verweilzeit gibt die mittlere Zeitdauer an, welche ein Partikel in einem Biogasreaktor bei eine kontinuierlichen Betriebsweise verweilt.

Gemäß einer anderen bevorzugten Ausgestaltung des erfindungsgemäßen Verfahrens werden die Richtwerte periodisch überprüft und die Zugabe der entsprechend ausgewählten enzymhaltigen Präparate entsprechend angepasst. Bevorzugt werden die Richtwerte zu Beginn der Enzymzugabe einmal wöchentlich, später einmal monatlich überprüft.

Gemäß einer bevorzugten Ausgestaltung des erfindungsgemäßen Verfahrens werden das oder die Merkmale, auf Basis derer in Verbindung mit den zugehörigen Richtwerten und Auswahlkriterien das oder die enzymhaltigen Präparate zur Verbesserung der Hydrolyse ermittelt werden, anhand bestimmter Regeln ausgewählt. Beispielsweise werden die beiden Merkmale Zähigkeit (Ausgestaltung II) und Spezifische Biogasbildung (Ausgestaltung III) nacheinander geprüft und die jeweiligen Richtwerte durch die Zugabe der ermittelten enzymhaltigen Präparate eingestellt. Die Beseitigung einer Abweichung der Viskosität der Biomasse in einem Biogasreaktor von dem bevorzugten Richtwert vermindert der Rühraufwand, fördert aber auch die spezifische Biogasbildung und damit die relative Substratausnutzung. Beides trägt zur Wirtschaftlichkeit des Biogasprozesses insgesamt bei. Falls nach Rückführung der Zähigkeit auf den Richtwert noch eine Abweichung der spezifischen Biogasbildung von deren Richtwert bestehen sollte, so kann diese mit einem geringeren Einsatz des gemäß des entsprechenden Auswahlkriteriums ermittelten enzymhaltigen Präparats beseitigt werden. Gemäß einer weiteren Ausgestaltung werden zum Beispiel zunächst die Aktivitäten von für die Hydrolyse relevanten Enzymen oder Gruppen von Enzymen an die jeweiligen Richtwerte gemäß Ausgestaltung I angepasst. Falls anschließend Abweichungen der Viskosität oder der spezifischen Biogasbildung von den jeweiligen Richtwerten noch bestehen sollten, werden diese gemäß der in den Ausgestaltungen II und III beschriebenen Ausführungen beseitigt. Dabei wird bevorzugt zuerst die Abweichung der Viskosität von dem betreffenden Richtwert beseitigt.

Gemäß einer weiteren bevorzugten Ausgestaltung des erfindungsgemäßen Verfahrens werden das oder die enzymhaltigen Präparate anhand mindestens eines Merkmals der Biomasse aus einem Biogasreaktor ausgewählt, welches mit einem oder mehreren der in den Ausgestaltungen I-III beschriebenen Merkmale korreliert. Unter "korreliert" wird hierbei verstanden, dass ein aus den Merkmalsdaten berechnetes Korrelationsmaß (beispielsweise der Korrelationskoeffizient nach Pearson) einen vorgegebenen Schwellenwert überschreitet. Beispielsweise kann anstelle der Viskosität der Trockenmassegehalt einer Biomasseprobe als Merkmal verwendet werden, und hierfür eine Richtwert theoretisch oder empirisch ermittelt werden, weil bekannt ist, dass die Viskosität einer Biomasseprobe mit der Trockenmassegehalt korreliert. Ferner hat sich überraschenderweise gezeigt, dass die Wirkung verschiedener enzymhaltiger Präparate auf die spezifische Biogasbildung aus Biomasseproben aus verschiedenen Biogasreaktoren mit bestimmten anderen Merkmalen der Biomasse korreliert. Beispielsweise korreliert die Wirkung verschiedener enzymhaltiger Präparate auf die spezifische Biogasbildung aus Biomasseproben mit dem Cellulosegehalt der Biomasseproben. Deshalb kann beispielsweise der Cellulosegehalt der Biomasse in einem Biogasreaktor mit Hilfe einer geeigneten Methode bestimmt und im Falle eines hohen gemessenen Cellulosegehalts der Biomasse, das oder die enzymhaltigen Präparate der Biomasse in dem Biogasreaktor zur Verbesserung der spezifischen Biogasbildung aus der Biomasse zugesetzt werden, bei denen die gemessene Korrelation einen bestimmten Schwellwert überschreitet oder maximal ist. Dabei kann die Korrelation je nach Merkmal positiv oder negativ sein. Bevorzugt werden die Merkmale der Biomasse aus der Gruppe der folgende Merkmale ausgewählt: Trockenmassegehalt, Gehalt an organischer Trockenmasse, Rohfasergehalt, NDF-Gehalt, ADF-Gehalt, ADL-Gehalt, Gehalt an Hemicellulosen, Gehalt an Cellulose, Gehalt an Pektinen, Gehalt an Lignin, Gehalt an Makro- und Spurenelementen, pH-Wert, elektrische Leitfähigkeit, Aktivitäten von Enzymen. Weiterhin bevorzugt werden die ermittelten Korrelationen für mehrere Merkmale miteinander verglichen und in Verbindung gebracht. Daraus können logische Regeln entwickelt werden, die festlegen, unter welchen Bedingungen ein oder mehrere enzymhaltige Präparate ausgewählt werden.

Gemäß einer anderen bevorzugten Ausgestaltung des erfindungsgemäßen Verfahrens werden die Enzyme aus der Liste folgender Enzyme, aus denen sich die enzymhaltigen Präparate zusammensetzen, bevorzugt aus folgender Gruppe von Enzymen ausgewählt: Cellobiohydrolase, beta-1,4-Endoglucanase, beta-1,4-Glucosidase, beta-1,4-Endoxylanase, beta-1,4-Xylosidase, beta-1,4(1,3)-Endoglucanase, Xyloglucan-beta-1,4-Endoglucanase, Exopolygalacturonase, Endopolygalacturonase, Pektinlyase, Pektatlyase, Rhamnogalacturonanlyase, beta-1,4-Endomannanase, beta-1,4-Endogalactanase, Mannosidase, Exoarabinase, Endoarabinase, Lichenase, Laminarinase, alpha-1,4-Galactosidase, beta-1,4-Galactosidase, alpha-Arabinofuranosidase, alpha-Xylosidase, alpha-Fucosidase, alpha-Glucuronidase, alpha-Rhamnosidase, Feruloylesterase, Acetylesterase, Methylesterase.

Gemäß einer bevorzugten Ausgestaltung des erfindungsgemäßen Verfahrens wird mindestens ein Metabolit oder Parameter, bezogen auf die Biomasse aus einem Biogasreaktor, der als Indikator für eine Limitierung durch die Hydrolyse dient, und mindestens ein Messverfahren für die Messung der Konzentration des mindestens einen Metaboliten in der Biomasse angegeben. Des Weiteren wird mindestens ein Richtwert für die Konzentration des mindestens einen Metaboliten oder Parameters in der Biomasse zur Verfügung gestellt. Gemäß dieser bevorzugten Ausgestaltung erfolgt eine Zugabe des einen oder der mehreren enzymhaltigen Präparate zu der Biomasse in einem Biogasreaktor nur, solange die in der Biomasse gemessene Konzentration des mindestens einen Metaboliten oder der gemessene Wert des mindestens einen Parameters unterhalb des Richtwertes liegt. Bevorzugt wird der Metabolit aus der Gruppe der folgenden Metabolite gewählt: Essigsäure, Propionsäure, Buttersäure, iso-Buttersäure. Die Konzentration des Metaboliten in der Biomasse kann mit Hilfe verschiedener Messmethoden ermittelt werden. Bevorzugt wird die Konzentration des Metaboliten in der Biomasse mit Hilfe des Verfahrens der Gaschromatografie ermittelt. Besonders bevorzugt wird Propionsäure als Metabolit verwendet. Ein bevorzugter Richtwert für den Gehalt an Propionsäure in der Biomasse ist 30 mg pro kg Frischmasse. Bevorzugt wird als Parameter der Gesamtsäuregehalt, ausgedrückt als Gehalt an Essigsäure, gewählt. Der bevorzugte Richtwert für den Gesamtsäuregehalt ist 1 g pro kg Frischmasse. Durch weitere Untersuchungen können weitere Erkenntnisse gewonnen werden, welche zu geeigneteren Metaboliten oder Parametern, besseren Messmethoden und genaueren Richtwerten für die Metabolite oder Parameter führen.

Gemäß einer bevorzugten Ausgestaltung des erfindungsgemäßen Verfahrens werden das oder die Enzympräparate direkt in die Biomasse in dem Biogasreaktor gegeben.

Gemäß einer weiteren bevorzugten Ausgestaltung des erfindungsgemäßen Verfahrens werden das oder die Enzympräparate täglich in einer gleichen Menge in die Biomasse gegeben. In einer anderen bevorzugten Ausgestaltung des erfindungsgemäßen Verfahrens werden das oder die enzymhaltigen Präparate in einer täglich variierenden Menge zugegeben. Bevorzugt werden das oder die enzymhaltigen Präparate zu Beginn der Enzymzugabe in höherer Menge und in der Folge abnehmender Menge der Biomasse in dem Bioreaktor zugegeben. Weiterhin bevorzugt werden das oder die enzymhaltigen Präparate in einer zeitlich oszillierenden Dosierung oder sonst einem definierten zeitlichen Dosierungsprofil der Biomasse zugegeben.

Gemäß einer Ausgestaltung des erfindungsgemäßen Verfahrens werden im Falle mehrerer ausgewählter enzymhaltiger Präparate diese gleichzeitig aber getrennt voneinander in die Biomasse in dem Bioreaktor gegeben. Gemäß einer weiteren Ausgestaltung werden mehrere ausgewählte enzymhaltige Präparate vor der Zugabe in den Biogasreaktor gemischt. Gemäß einer anderen Ausgestaltung werden die ausgewählten enzymhaltigen Präparate zu verschiedenen Zeiten in die Biomasse in dem Biogasreaktor gegeben. Bevorzugt wird hierfür ein definiertes zeitliches Dosierungsschema vorgegeben. Besonders bevorzugt orientiert sich dieses Dosierungsschema an Merkmalen der Betriebsführung der Biogasanlage, beispielsweise an der Fütterung der Biogasanlage mit Inputstoffen und/oder an Rührereignissen und/oder der Zugabe anderer prozessfördernder Hilfsmittel.

In einer anderen bevorzugten Ausgestaltung des erfindungsgemäßen Verfahrens werden das oder die Enzympräparate gemeinsam mit anderen Prozesshilfsmitteln, bevorzugt mit spurenelementhaltigen Präparaten und/oder mit Präparaten der Biomasse in dem Bioreaktor zugegeben, welche Ammonium binden und den Ammoniumgehalt senken. Durch die Beschleunigung der Hydrolase kann einer der nachfolgenden Prozessschritte des Biogasprozesses unbeabsichtigt limitierend wirken, insbesondere die Methanogenese. Durch die gleichzeitige Zugabe spurenelementhaltiger Präparate kann dieser Effekt verhindert werden. Durch die Bindung von Ammonium kann die Stabilität von Enzymen in der Biomasse erhöht werden.

Gemäß einer weiteren bevorzugten Ausgestaltung des erfindungsgemäßen Verfahrens können die enzymhaltigen Präparate in fester oder flüssiger Form in die Biomasse in dem Biogasreaktor gegeben werden. Bevorzugt werden die Präparate in fester Form zugegeben.

Gemäß einer weiteren bevorzugten Ausgestaltung des erfindungsgemäßen Verfahrens werden das oder die emzymhaltigen Präparate zusammen mit Wirkstoffen der Biomasse zugegeben, welche die katalytische Wirkung und die Bioverfügbarkeit der Enzyme fördern. Bevorzugt werden die Wirkstoffe zum Beispiel aus der Gruppe folgender Wirkstoffe ausgewählt: spezifische, für die katalytische Wirkung bestimmter Enzyme notwendige Metalle, oberflächenaktive Stoffe und Chelatbildner, durch welche inhibierende Ionen komplexiert werden oder Oxalsäure.

Gemäß einer weiteren bevorzugten Ausgestaltung des erfindungsgemäßen Verfahrens werden das oder die emzymhaltigen Präparate zusammen mit Wasser der Biomasse so zugegeben, dass die Wasseraktivität in der Biomasse einen vorgegebenen Schwellenwert übersteigt oder erreicht. Hierdurch wird die Beweglichkeit der Enzymmoleküle (Diffusionskoeffizient) erhöht und die katalytische Wirkung verbessert.

Gemäß einer anderen bevorzugten Ausgestaltung des erfindungsgemäßen Verfahrens werden die Enzyme dadurch vor Inaktivierung durch Wärme, Proteasen oder sonstige Einflüsse seitens der Zusammensetzung und der physikalischen Eigenschaften der Biomasse geschützt, dass sie in einer geeigneten Weise formuliert werden, beispielweise mit Hilfe eines Coating-Verfahrens gekapselt werden. In einer besonderen Ausführung dieser Ausgestaltung dient das Coating der Verlangsamung der Freisetzung der Enzyme aus den Bestandteilen des enzymhaltigen Präparats in die Biomasse.
Beispiele der Erfindung werden nachfolgend anhand von Abbildungen erläutert. In den Abbildungen zeigen:
Abbildung 1:
   Relative Aktivitäten hydrolytischer Enzyme bzw. Enzymgruppen (Ordinate), gemessen in 76 Proben aus Biogasreaktoren von Biogasanlagen (Abszisse). Der für jedes Enzym bzw. Enzymgruppe höchste gemessene Wert innerhalb der Stichprobe wurde gleich 1 gesetzt.
Abbildung 2:
   Relativer Verzuckerungsgrad der Inputstoffe Maissilage und Grassilage (Ordinate) nach 24-ständiger Inkubation mit 10 verschiedenen enzymhaltigen Präparaten (E1-E10, Abszisse). Für jeden Inputstoff wurde der höchste erzielte Verzuckerungsgrad gleich 1 gesetzt.
Abbildung 3:
   Enzymprofile der (siehe Abbildung 2) eingesetzten enzymhaltigen Präparate, dargestellt als Netzgrafik (C=Cellulase, X=Xylanase, P=Pektinase, A=a-L-Arabinofuranosidase). Dabei wurde für jedes Enzym bzw. jede Enzymgruppe die relative Aktivität aufgetragen, wobei die innerhalb der 10 enzymhaltigen Präparate höchste gemessene Aktivität auf dem jeweiligen Eckpunkt der Netzgrafik liegt und den Wert 1 besitzt.
Abbildung 4:
   Profil von Zuckern und Zuckersäuren der Biomasse aus einem Biogasreaktor, dargestellt als Netzgrafik (G=Glucose, X=Xylan, U=Galacturonsäure, A=Arabinose). Dabei wurden die Gehalte in relativen Einheiten aufgetragen, wobei der höchste Gehalt = 1 gesetzt wurde und auf dem entsprechenden Eckpunkt der Netzgrafik liegt.
Abbildung 5:
   Viskosität in dPas (Ordinate) in der Biomasse aus 76 Biogasreaktoren von Biogasanlagen (Abszisse).
Abbildung 6:
   Relative Abnahme der Viskosität in % (Ordinate) in drei verschiedenen Biomasseproben aus Biogasreaktoren nach Zugabe verschiedener enzymhaltiger Präparate (Abszisse) nach 140-stündiger Inkubation bei 40°C im Vergleich zu einer Kontrolle ohne Zugabe eines enzymhaltigen Präparats, gemessen mit Hilfe eines Rotationsviskosimeters nach vorheriger Zerkleinerung der Biomasse.
Abbildung 7:
   Viskosität in der Biomasse aus den Fermentern von Linie 1 und 2 in dPas (Ordinate) in einem Praxisversuch an einer Biogasanlage über mehrere Wochen (Abszisse). Die Viskosität wurde mit Hilfe eines Rotationsviskosimeters nach 144-stündiger Inkubation bei 40°C gemessen. Vorher wurden die Proben aus den Fermentern mittels eines Thermomixers zerkleinert. Mit Beginn der Woche 1 wurde die Fütterung in beiden Linien auf eine Mischung aus 60 % Roggenganzpflanzensilage und 40 % Maissilage umgestellt. Mit Beginn der Woche 9 wurde in Linie 1 das enzymhaltige Präparat E3 und in Linie 2 das enzymhaltige Präparat E5 (siehe Beispiel 4), beide in gleiche Mengen, täglich dosiert. Der Richtwert für die Viskosität liegt bei 20 dPas (siehe Beispiel 4).
Abbildung 8:
   Relativer spezifischer Mehrbiogasbildung (Ordinate) aus der Biomasse aus 76 Biogasreaktoren von Biogasanlagen (Abszisse).
Abbildung 9:
   Relativer spezifischer Biogasmehrertrag in % (Ordinate) in drei verschiedenen Biomasseproben aus Biogasreaktoren nach Zugabe verschiedener enzymhaltiger Präparate (Abszisse) nach 140-stündiger Inkubation bei 40 °C im Vergleich zu einer Kontrolle ohne Zugabe eines enzymhaltigen Präparats, gemessen mit Hilfe des ANKOM-Systems.
Abbildung 10:
   Größenverteilung der Cellulaseaktivität in Biomasse-Proben aus verschiedenen Biogasreaktoren.
Abbildung 11:
   Vergleich der Mittelwerte der Cellulaseaktivität bei guter und schlechter relativer Substratausnutzung.
Abbildung 12:
   Größenverteilung der Xylanaseaktivität in Biomasse-Proben aus verschiedenen Biogasreaktoren.
Abbildung 13:
   Vergleich der Mittelwerte der Xylanaseaktivität bei guter und schlechter relativer Substratausnutzung.
Abbildung 14:
   Größenverteilung der Gesamtesteraseaktivität in Biomasse-Probenaus verschiedenen Biogasreaktoren.
Abbildung 15:
   Vergleich der Mittelwerte der Gesamtesteraseaktivität bei guter und schlechter relativer Substratausnutzung.
Abbildung 16:
   Größenverteilung der Lipaseaktivität in Biomasse-Proben aus verschiedenen Biogasreaktoren.
Abbildung 17:
   Vergleich der Mittelwerte der Lipaseaktivität bei guter und schlechter relativer Substratausnutzung.
Abbildung 18:
   Kommulierte relative Zunahme der Enzymaktivitäten in Biomasse-Proben aus verschiedenen Biogasreaktoren (schwarz: Cellulaseaktivität, Maissilage; dunkelgrau: Cellulaseaktivität, Grassilage; hellgrau: Xylanaseaktivität, Maissilage und weiß: Xylanaseaktivität, Grassilage).
Abbildung 19:
   Größenverteilung der relativen Zunahme der Cellulaseaktivität in Biomasse-Proben aus verschiedenen Biogasanlagen nach Zugabe von Maissilage.
Abbildung 20:
   Größenverteilung der relativen Zunahme der Cellulaseaktivität in Biomasse-Proben aus verschiedenen Biogasanlagen nach Zugabe von Grassilage.
Abbildung 21:
   Größenverteilung der relativen Zunahme der Xylanaseaktivität in Biomasse-Proben aus verschiedenen Biogasanlagen nach Zugabe von Maissilage.
Abbildung 22:
   Größenverteilung der relativen Zunahme von Xylanaseaktivität in Biomasse-Proben aus verschiedenen Biogasanlagen nach Zugabe von Grassilage.
Abbildung 23:
   Größenverteilung der spezifischen Biogasbildung aus Biomasseproben verschiedener Biogasanlagen.
Abbildung 24:
   Relativer Biogas-Mehrertrag aus verschiedenen Biomasse-Proben aus Biogasreaktoren nach 24 h Inkubation nach Zugabe verschiedener enzymhaltiger Präparate.
Abbildung 25:
   Größenverteilung der Zähigkeit der Biomasse-Proben aus verschiedenen Biogasanlagen, gemessen mit dem Drehmomentviskosimeter.
Abbildung 26:
   Relative Verflüssigung verschiedener Biomasse-Proben aus Biogasreaktoren nach 140 h Inkubation nach Zugabe verschiedener enzymhaltiger Präparate.
Abbildung 27:
   Aus Maissilage gebildete Menge Biogas (schwarze Rauten) und Cellulaseaktivität (rote Quadrate) aus verschiedenen Biomasse-Proben aus Biogasreaktoren nach 48 h Inkubation, wobei die Biogaserträge der Größe nach geordnet wurden und der Schreiben-Gehalt der Biomasse-Probe auf 5 % eingestellt wurde. Für den Vergleich wurde der annähernd lineare Bereich der Größenverteilung der Biogaserträge gewählt.
Abbildung 28:
   Aus Grassilage gebildete Menge Biogas (schwarze Rauten) und Cellulaseaktivität (rote Quadrate) aus verschiedenen Biomasse-Proben aus Biogasreaktoren nach 48 h Inkubation, wobei die Biogaserträge der Größe nach geordnet wurden und der TS-Gehalt der Biomasse-Probe auf 5 % eingestellt wurde. Für den Vergleich wurde der annähernd lineare Bereich der Größenverteilung der Biogaserträge gewählt.
Abbildung 29:
   Aus Maissilage gebildete Menge Biogas nach 48 h Inkubation in Abhängigkeit von der Relativen Substratausnutzung. Der TS-Gehalt der Biomasse-Probe wurde auf 5 % eingestellt.
Abbildung 30:
   Aus Grassilage gebildete Menge Biogas nach 48 h Inkubation in Abhängigkeit von der Relativen Substratausnutzung. Der TS-Gehalt der Biomasse-Probe wurde auf 5 % eingestellt.
Abbildung 31:
   Korrelation der spezifischen Biogasbildung aus Biomasse-Proben nach 140 h Inkubation und der in den Biomasse-Proben mit einem Drehmomentviskosimeter gemessenen Zähigkeit.
Abbildung 32:
   Korrelationen der Wirkung verschiedener enzymhaltiger Präparate auf die relative Steigerung des Biogasertrags gegenüber der Kontrolle ohne Zusatz eines Präparats nach 140 h Inkubation mit dem Cellulose-Gehalt der Biomasse.
Abbildung 33:
   Korrelationen der Wirkung verschiedener enzymhaltiger Präparate auf die Verbesserung des Drehmoments, gemessen mit dem Drehmomentviskosimeter, gegenüber der Kontrolle ohne Zusatz eines Präparats nach 140 h Inkubation mit dem Hemicellulose-Gehalt der Biomasse.

### Beispiel 1 zur Ausgestaltung I des erfindungsgemäßen Verfahrens

Aus den in Abbildung 1 dargestellten relativen Aktivitäten der Enzyme oder Enzymgruppen Cellulase, Xylanase, Pektinase und α-L-Arabinofuranosidase wird die empirische Häufigkeitsverteilung mit einer Klassenbreite von 0,5 bestimmt. Im Anschluss werden aus den Häufigkeitsverteilungen jeweils die 75 %-Quantile ermittelt. Diese betragen für die in Abbildung 1 dargestellten Messergebnisse: Cellulase 1,66 U pro g Trockensubstanz; Xylanase 2,95 U pro g Trockensubstanz; Pektinase 3,50 U pro g Trockensubstanz; α-L-Arabinofuranosidase 0,54 U pro g Trockensubstanz. Diese Werte werden als Richtwerte genommen.

Dabei werden die Aktivitäten der Enzyme bzw. Enzymgruppen in den Biomasseproben aus den Biogasreaktoren nach folgenden Methoden bestimmt:

### Probenvorbereitung:

Die Biomasseprobe aus dem Bioreaktor wird bei -80°C eingefroren. Vor der Durchführung der Messung wird die Biomasseprobe über Nacht im Kühlschrank auftaut. Je 10g der Probe werden in Falcon-Tubes eingewogen, 40 ml Aqua. dest. zugegeben (1:5 Verdünnung) und die Suspension 5 min in einem Vortex-Mischer homogenisiert. Anschließend wird der Inhalt auf 2-mL-Reaktionsgefäße verteilt und bei 13.000 rpm 10 min zentrifugiert. Der Überstände (=Enzymlösungen) werden in ein neues Reaktionsgefäß überführt und bis zur Messung auf Eis kühl gehalten.

### Messung der Cellulaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 150 µL En-zymlösung 350 µl Substrat (1% Carboxymethylcellulose in 250 mM Kaliumphosphatpuffer, pH 7.0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Glucose-Standards erstellt wird. 1 U = 1 µmol Glucose pro Minute bei 40°C und pH=7.

### Messung der Xylanaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 150 µL Enzymlösung 350 µl Substrat (1,5% Xylan in 250mM Kaliumphosphatpuffer, pH 7,0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Xylose-Standards erstellt wird. 1 U = 1 µmol Xylose pro Minute bei 40°C und pH=7,0.

### Messung der Pektinaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 100 µL Enzymlösung 200 µl Substrat (1,2% Polygalacturonsäure in 250mM Kaliumphosphatpuffer, pH 7,0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Galacturonsäure-Standards erstellt wird. 1 U = 1 µmol Galacturonsäure pro Minute bei 40°C und pH=7,0.

### Messung der α-L-Arabinofuranosidaseaktivität:

Der Assay wird in Doppelbestimmung durchgeführt. 450 µl Na-Acetat-Puffer, 0.05M, pH 4.0 werden mit 50 µl Substrat-Lösung in ein 1,5 ml Reaktionsgefäß gegeben und 5 min bei 50°C im Thermomixer vorgewärmt. Es werden 10 µl Probe hinzufügt, 15 min bei 50°C im Thermomixer inkubiert, 500 µl Stopp-Lösung zugegeben und gemischt. Der Testansatz wird in eine Halbmikroküvette überführt und bei E (405 nm) gegen Puffer gemessen. Die Nullprobe ist vor der Zugabe des Substrates bereits mit der Stopplösung zu versetzen. 1 U = 1 µmol Arabinose pro Minute bei 50°C und pH=4,0.

In einer Probe aus einem Biogasreaktor einer Biogasanlage wurden die Aktivitäten von Cellulase, Xylanase, Pektinase und α-L-Arabinofuranosidase gemäß der beschriebenen Methoden bestimmt. Die Aktivitäten betrugen: Cellulase 2,20 U pro g Trockensubstanz; Xylanase 2,40 U pro g Trockensubstanz; Pektinase 0,72 U pro g Trockensubstanz; α-L-Arabinofuranosidase 0,55 U pro g Trockensubstanz. Damit lagen die gemessenen Aktivitäten von Xylanase und Pektinase unter den oben ermittelten Richtwerten.

Aus einer Gruppe von 6 sonst gleichartigen enzymhaltigen Präparaten wurden zwei Präparate ausgewählt, von denen das eine die höchste Xylanaseaktivität, das andere die höchste Pektinaseaktivität aufwies. Diese beiden Präparate wurden in einem solchen Verhältnis gemischt, dass das Mischpräparat im Vergleich zu den nicht ausgewählten 4 Präparaten die höchste Xylanase- und zugleich die höchste Pektinaseaktivität aufwies.

Dieses Präparat wurde der beprobten Biogasanlage zugegeben.

### Beispiel 2 zur Ausgestaltung I des erfindungsgemäßen Verfahrens

Richtwerte für die Aktivitäten zellwandabbauender Enzyme oder Enzymgruppen, hier expliziert am Beispiel der Enzyme bzw. Enzymgruppen Cellulase, Xylanase, Pektinase und α-L-Arabinofuranosidase und für den Fall, dass eine Biogasanlage überwiegend Maissilage einsetzt, lassen sich beispielsweise wie folgt gewinnen:

Das in Abbildung 3 dargestellte Enzymprofil des enzymhaltigen Präparats E8, welches den höchsten relativen Verzuckerungsgrad an Maissilage erzielt, zeigt folgende relative Aktivitäten, gemessen an der Aktivität der Xylanase, die den Wert 1 besitzt: Cellulase 0,5; Xylanase 1,0; Pektinase 0,5; α-L-Ara¬bino¬furanosidase 0,9.

Zur Ableitung der inputstoffspezifischen Richtwerte werden diese relativen Aktvitäten beispielsweise mit dem Richtwert für Xylanase multipliziert, welcher in Beispiel 1 angegeben ist: 2.95 U pro g Trockensubstanz. Dies geschieht deshalb, weil Xylanase die höchste relative Aktivität der 4 für das enzymhaltige Präparat E8 gemessenen Enzymaktivitäten besitzt.

Hieraus ergeben sich die für den Abbau von Maissilage geltenden Richtwerte wie folgt:
Cellulase: 2,95 U/gTS x 0,5 = 1,48 U/gTS
Xylanase: 2,95 U/gTS
Pektinase: 2,95 U/gTS x 0,5 = 1,48 U/gTS
α-L-Arabinofuranosidase: 2,95 U/gTS x 0,9 = 2,66 U/gTS

Für den Fall, dass innerhalb des Enzymprofils eines enzymhaltigen Präparats, welches sich am besten für den Abbau eines Inputstoffs erwiesen hat, kein Enzym bzw. keine Enzymgruppe die relative Aktivität 1 besitzt, wird die höchste der für dieses Präparat gemessenen relativen Aktivitäten gleich 1 gesetzt und die relativen Aktivitäten der anderen Enzyme oder Enzymgruppen entsprechend ihres Verhältnisses zu der höchsten gemessenen Aktivität entsprechend angepasst.

Für den Fall, dass eine Biogasanlage die Inputstoffe Maissilage und Grassilage zu 40% bzw. 60%, bezogen auf die Gesamttrockenmasse der Inputstoffe, einsetzt, werden die inputstoffspezifischen Richtwerte wie folgt bestimmt:
Das in Abbildung 3 dargestellte Enzymprofil des enzymhaltigen Präparats E8, welches den höchsten relativen Verzuckerungsgrad an Maissilage erzielt, zeigt folgende relative Aktivitäten, gemessen an der Aktivität der Xylanase, die den Wert 1 besitzt: Cellulase 0,5; Xylanase 1,0; Pektinase 0,5; α-L-Arabinofuranosidase 0,9. Das in Abbildung 3 dargestellte Enzymprofil des enzymhaltigen Präparats E7, welches den höchsten relativen Verzuckerungsgrad an Grassilage erzielt, zeigt folgende relative Aktvitäten, gemessen an der Aktivität der Cellulase, die den Wert 1 besitzt: Cellulase 1,0; Xylanase 0,7; Pektinase 0,4; α-L-Arabinofuranosidase 0,7.

Die relativen Aktivitäten der enzymhaltigen Präparate E7 und E8 werden gemäß der Anteile an Maissilage bzw. Grassilage an der Gesamttrockenmasse der Inputstoffe linear kombiniert:
Cellulase: 0,5 x 0,4 + 1,0 x 0,6 = 0,80
Xylanase: 1,0 x 0,4 + 0,7 x 0,6 = 0,82
Pektinase: 0,5 x 0,4 + 0,4 x 0,6 = 0,44
α-L-Arabinofuranosidase: 0,9 x 0,4 + 0,7 x 0,6 = 0,78

Hierbei ergibt sich für Xylanase der höchste Faktor (0,82). Anschließend die werden so erhaltenen Faktoren deshalb mit Bezug auf Xylanase auf den Wert 1 normiert:
Cellulase: 0,98
Xylanase: 1,00
Pektinase: 0,54
α-L-Arabinofuranosidase: 0,95

Zuletzt werden die normierten Faktoren mit dem Richtwert für Xylanase (0,95 U/gTS) wie weiter oben im Beispiel multipliziert. Auf diese Weise erhält man die folgenden inputstoffspezifischen Richtwerte für die obige Inputstoffmischung:
Cellulase: 2,95 U/gTS x 0,98 = 2,89 U/gTS
Xylanase: 2,95 U/gTS
Pektinase: 2,95 U/gTS x 0,54 = 1,59 U/gTS
α-L-Arabinofuranosidase: 2,95 U/gTS x 0,95 = 2,80 U/gTS

### Beispiel 3 zur Ausgestaltung I des erfindungsgemäßen Verfahrens

Stoffspezifische Richtwerte für die Aktivitäten zellwandabbauender Enzyme oder Enzymgruppen, hier expliziert am Beispiel der Enzyme bzw. Enzymgruppen Cellulase, Xylanase, Pektinase und α-L-Arabinofuranosidase lassen sich beispielsweise wie folgt gewinnen:
Das in Abbildung 4 dargestellte Stoffprofil der Biomasse aus einem Biogasreaktor zeigt die folgenden relativen Zucker- bzw. Zuckersäurengehalte der Biomasse:
Glucose: 0,6
Xylose: 1,0
Galacturonsäure: 0,1
Arabinose: 0,3

Diese relativen Gehalte werden mit dem Richtwert für Xylanase gemäß Beispiel 1 (2,95 U/gTS) multipliziert. Dies geschieht deshalb, weil Xylose als Bestandteil von Xylan, welches durch Xylanase abgebaut wird, den höchsten relativen Gehalt der vier untersuchten stofflichen Bestandteile der Biomasse in dem Biogasreaktor besitzt. Hieraus ergeben sich die stoffspezifischen Richtwerte für die Enzyme Cellulase, Xylanase, Pektinase und α-L-Arabinofuranosidase:
Cellulase: 2,95 U/gTS x 0,6 = 1,77 U/gTS
Xylanase: 2,95 U/gTS
Pektinase: 2,95 U/gTS x 0,1 = 0,30 U/gTS
α-L-Arabinofuranosidase: 2,95 U/gTS x 0,3 = 0,90 U/gTS

### Beispiel 4 zur Ausgestaltung II des erfindungsgemäßen Verfahrens

Aus der in Abbildung 5 dargestellten Verteilung der Viskosität wird die empirische Häufigkeitsvertei-lung mit der Klassenbreite von 10 dPas bestimmt. Im Anschluss wird aus der Häufigkeitsverteilung das 45%-Quantil ermittelt. Dieses beträgt für die in Abbildung 5 dargestellten Messergebnisse: 20 dPas. Dieser Wert wird als Richtwert genommen.

In einer Versuchsbiogasanlage (750 kW) mit zwei parallelen Linien wurde eine Mischung aus 40% Maissilage und 60% Roggenganzpflanzensilage über einen Zeitraum von mehreren Wochen gefüttert und die Viskosität in beiden Fermentern (Biogasreaktoren) wöchentlich gemessen (siehe Abbildung 7).

Nach Erreichen einer Viskosität von über 40 dPas (nach 7 Wochen) wurde eine Mischprobe aus beiden Fermentern mit 6 verschiedenen enzymhaltigen Präparaten (E1-E6) inkubiert und die Viskosität nach 144 stündiger Inkubation bei 40°C im Vergleich zu einer Kontrolle ohne Zugabe eines Enzympräparats mit Hilfe eines Rotationsviskosimeters gemessen. Zuvor wurde die Probe mit Hilfe eines Thermomixers zerkleinert. Dabei ergaben sich für die 6 enzymhaltigen Präparate folgende relative Änderungen der Viskosität im Vergleich zur Kontrolle:
E1: -9%
E2: +2%
E3: -28%
E4: -4%
E5: -18%
E6: -12%

Mit Beginn der 9. Woche wurde in Linie 1 das enzymhaltige Präparat E3 und in Linie 2 das enzymhaltige Präparat E5 in gleichen Mengen zugegeben. Abbildung 7 zeigt, dass in Linie 1 der Richtwert in Höhe von 20 dPas 3 Wochen nach Zugabe der Präparate unterschritten wurde. In Linie 2 war der Richtwert 5 Wochen nach Zugabe der Präparate noch nicht erreicht.

### Beispiel 5 zur Ausgestaltung III des erfindungsgemäßen Verfahrens

Aus der in Abbildung 8 dargestellten Verteilung der relativen spezifischen Biogasbildung wird die empirische Häufigkeitsverteilung mit einer Klassenbreite von 0,5 bestimmt. Im Anschluss wird aus der Häufigkeitsverteilung das 75%-Quantil ermittelt. Dieses beträgt für die in Abbildung 8 dargestellten Messergebnisse: 0,54. Dieser Wert wird als Richtwert genommen.

In der Biomasse aus einem Biogasreaktor wurde eine Probe entnommen und die aus der Probe gebildete relative spezifische Biogasmenge mit Hilfe des ANKOM-Systems nach 140-stündiger Inkubation bei 40°C bestimmt. Dieser Wert betrug, gemessen an der maximalen Biogasbildung gemäß der Verteilung in Abbildung 8, 0,42 und lag unterhalb des Richtwertes.

Dieselbe Biomasseprobe wurde mit 6 verschiedenen enzymhaltigen Präparaten (E1-E6) inkubiert und die relative spezifische Mehrbiogasbildung nach 140 stündiger Inkubation bei 40°C, ebenfalls mit Hilfe des ANKOM-Systems, gemessen. Dabei ergaben sich für die 6 enzymhaltigen Präparate folgenden prozentualen spezifischen Biogasmehrerträge:
E1: 5%
E2: 34%
E3: 1%
E4: 20%
E5: 16%
E6: 8%

Das Präparat E2 wurde der beprobten Biogasanlage zugegeben.

In Anhang 2 sind weitere Varianten und Ausführungsbeispiele der Erfindung angegeben.

### Anhang 2

### Nachfolgend sind weitere Varianten und Ausführungsbeispiele der Erfindung angegeben:

Das erfindungsgemäße Verfahren zur Erzeugung von Biogas in einem Biogasreaktor umfasst die folgenden Schritte:
1) Mindestens ein quantitatives Defizitmerkmal, bezogen auf die Biomasse aus einem bestimmten Biogasreaktor, wird angegeben.
2) Mindestens ein Messverfahren für die Messung des mindestens einen Defizitmerkmals wird angegeben.
3) Mindestens ein Richtwert für das mindestens eine Defizitmerkmal wird ermittelt und zur Verfügung gestellt.
4) In dem Biogasreaktor wird Biogas erzeugt.
5) Der Wert des mindestens einen Defizitmerkmals in der Biomasse aus dem Biogasreaktor wird mit Hilfe des mindestens einen Messverfahrens ermittelt.
6) Mindestens ein quantitatives Auswahlkriterium für die Auswahl mindestens eines enzymhaltigen Präparates aus einer gegebenen Gruppe von mindestens zwei enzymhaltigen Präparaten wird zur Verfügung gestellt.
7) Falls der ermittelte Defizitmerkmalswert in der Biomasse von dem mindestens einen ermittelten und zur Verfügung gestellten Richtwert abweicht, werden das oder die gemäß des mindestens einen Auswahlkriteriums ausgewählten enzymhaltigen Präparate mindestens in einer solchen Menge oder über einen solchen Zeitraum der Biomasse in dem Biograsreaktor zugegeben, bis die gemessene Abweichung verschwindet.

Bei dem erfindungsgemäßen Verfahren wird der Aufwand für die Anwendung von Enzymen minimiert wird, indem die Auswahl und eingesetzte Menge eines oder mehrerer enzymhaltiger Präparate aus einer gegebenen Gruppe von mindestens zwei enzymhaltigen Präparaten auf der Basis der empirischen Ermittlung bestimmter quantitativer Defizitmerkmale der Biomasse in Verbindung mit bestimmten Auswahlkriterien und gegebenenfalls unter Berücksichtigung von Zusammenhängen zwischen verschiedenen Defizitmerkmalen untereinander oder zwischen Defizitmerkmalen und anderen Merkmalen der Biomasse so erfolgt, dass die eingesetzte Gesamtmenge an enzymhaltigem Präparat oder enzymhaltigen Präparaten für einen oder mehrere quantifizierbare Verbesserungsschritte des oder der Defiziterkmale minimal ist. Überraschenderweise hat sich nämlich gezeigt, dass sich die Biogaserzeugung in einem bestimmten Biogasreaktor dann besonders effizient gestalten lässt, wenn Richtwerte für solche Defizitmerkmale eingehalten werden, die in direktem kausalen Zusammenhang mit der Qualität der Hydrolyse (als Mitursache oder Folge der Qualität der Hydrolyse) stehen und die sich negativ auf den Gesamtprozess auswirken und die Einstellung dieser Richtwerte durch die Zugabe eines oder mehrerer enzymhaltiger Präparate aus einer gegebenen Gruppe von Präparaten so erfolgt, dass die zugelegte Gesamtmenge an dem oder den enzymhaltigen Präparaten minimal ist. Dies wiederum wird dadurch erreicht, dass zu den Defizitmerkmalen jeweils geeignete quantitative Auswahlkriterien für die Auswahl des oder der Präparate zur Verfügung gestellt werden. Dabei wird unter einem bestimmten Biogasreaktor vorzugsweise ein Biogasreaktor einer gegebenen Bauart und Ausführung verstanden, der mit gegebenen Inputstoffen, die sich im Laufe des Betriebs auch ändern können, betrieben und in dem ein Biogasverfahren durchgeführt wird. Ein Biogasreaktor kann insbesondere auch ein Behälter zur Vorhydrolyse, ein Fermenter, ein Nachgärer oder ein Endlager sein. Es versteht sich, dass ein Biogasreaktor in eine Biogasanlage integriert ist, die insbesondere Einrichtungen zur Vorbehandlung der Inputstoffe, zur Einbringung von Inputstoffen in den Biogasreaktor, Rohrleitungen für den Transport von Biomasse und Biogas, Pumpen, Biogasreinigungssysteme und Blockheizkraftwerke umfassen kann. Unter einem enzymhaltigen Präparat wird vorzugsweise eine Zusammensetzung aus einem oder mehreren Enzymen mit oder ohne einen oder mehrere Trägerstoffe, Stabilisatoren, Konservierungsstoffe oder andere technische Hilfsstoffe in fester oder flüssiger Form verstanden. Unter einem quantitativen Auswahlkriterium wird eine Regel verstanden, nach der bezogen auf ein oder mehrere messbare Merkmale (dies können ein oder mehrere Defizitmerkmale und/oder andere Merkmale sein, oder Kombinationen von Defizitmerkmalen mit anderen Merkmalen oder Defizitmerkmalen oder anderen Merkmalen untereinander) die Auswahl des einen oder der mehreren enzymhaltigen Präparate so erfolgt, dass bezüglich dieser Merkmale deren Ausprägung maximal oder minimal wird oder bestimmte Schwellenwerte erreicht oder über- oder unterschritten werden. Es ist anzunehmen, dass durch weitere Untersuchungen weitere Erkenntnisse gewonnen werden können, welche die Bereitstellung weiterer Defizitmerkmale und Messmethoden für die Defizitmerkmale, genauere Richtwerte und besserer Auswahlkriterien zur Auswahl der am besten geeigneten enzymhaltigen Präparate ermöglichen.
Unter quantitativen Defizitmerkmalen werden hier messbare Merkmale wie zum Beispiel die Aktivitäten für die Hydrolyse relevanter Enzyme, die Fähigkeit zur Bildung von Enzymen (gemessen an einer Zunahme der Aktivitäten) in der Biomasse nach einer Änderung der Betriebsweise des Bioreaktors (beispielsweise durch eine Änderung der Art oder Menge der Inputstoffe in den Biogasreaktor zugegebenen Inputstoffe), die Zähigkeit der Biomasse oder die spezifische Biogasbildung aus der Biomasse verstanden. Unter der Aktivität eines Enzyms wird hier ein Maß für die Umsatzgeschwindigkeit der durch dieses Enzym katalysierten biochemischen Reaktion verstanden, bezogen auf ein oder mehrere Substrate und gemessen mit einer geeigneten Methode. Unter dem Begriff Zähigkeit werden hier rheologische Eigenschaften, wie zum Beispiel die Viskosität, zusammengefasst, welche mit einer geeigneten Methode in der Biomasse gemessen werden können. Unter spezifischer Biogasbildung wird beispielsweise die mit oder ohne Zugabe eines oder mehrerer Inputstoffe aus der Biomasse in einer bestimmten Zeit gebildete Menge an Methan verstanden, wie sie mit einer geeigneten Messapparatur ermittelt werden kann, bezogen auf einen Parameter der Biomasse (beispielsweise den Gehalt an organischer Trockenmasse).

### Ausgestaltung I des erfindungsgemäßen Verfahrens: Defizitmerkmal sind die Aktivitäten für die Hydrolyse relevanter Enzyme:

Gemäß einer bevorzugten Ausgestaltung I des erfindungsgemäßen Verfahrens ist das quantitative Defizitmerkmal die Aktivität mindestens eines für die Hydrolyse maßgeblichen Enzyms oder einer maßgeblichen Gruppe von Enzymen. Diese Ausgestaltung des Verfahrens geht von der überraschenden Erkenntnis aus, dass die Biogaserzeugung in einem Biogasreaktor dann besonders effektiv ist, wenn die Aktivität mindestens eines für die Biogaserzeugung maßgeblichen Enzyms oder einer maßgeblichen Gruppe von Enzymen einen Richtwert einhält. Aktivitäten für Beispiele relevanter Enzyme und Gruppen von Enzymen wurden durch Untersuchungen an Praxisanlagen ermittelt. Überraschenderweise hat eine Reihenuntersuchung von 55 Biomasse-Proben aus Biogasreaktoren verschiedener Biogasanlagen gezeigt, dass die Aktivitäten gelöster hydrolytischer Enzyme bzw. Gruppen von Enzymen in den Gärschlämmen erheblichen Schwankungen unterworfen sind. Dies zeigen die Abbildungen 1, 3, 5 und 7 in Anhang 1 für die gelösten Fraktionen der Enzymgruppen Cellulase, Xylanase, Gesamtesterasen und Lipase. Abhängig von der Enzymgruppe, können die gemessenen Aktivitäten um mehr als eine Größenordnung schwanken. Die Verteilungen der Enzymaktivitäten sind also durch eine erhebliche Varianz charakterisiert. Beispielsweise liegt der kleinste Wert der Cellulaseaktivität in den untersuchten Biomasse-Proben bei 0,17 U/gTS, der größte Wert bei 2,24 U/gTS. Der Mittelwert der empirischen Verteilung der Cellulaseaktivität liegt bei 1,28 U/gTS, der Median bei 1,26 U/gTS. Ähnliche Verteilungen ergeben sich auch für weitere Enzyme und Gruppen von Enzymen. Überraschenderweise verteilen sich die Enzymaktivitäten in den Biomasse-Proben nicht gleichsinnig, das heißt gemessen an den gefundenen Maximalwerten der empirischen Verteilungen kann die Aktivität eines bestimmten Enzyms oder einer Gruppe von Enzymen niedrig, die eines anderen Enzyms oder einer Gruppe von Enzymen hoch sein (siehe dazu auch das Beispiel 1). Ebenso überraschend hat sich im Rahmen der Untersuchung gezeigt, dass ein Zusammenhang zwischen den Aktivitäten der untersuchten Enzyme bzw. Enzymgruppen in den Biomasse-Proben aus den Biogasreaktoren der beprobten Biogasanlagen und der relativen Substratausnutzung besteht. Dies zeigen die Abbildungen 2, 4, 6 und 8 in Anhang 1. Die relative Substratausnutzung ist definiert als diejenige Menge an Methan, welche aus den eingesetzten Inputstoffen gegenüber der erwartbaren Menge an Methan und relativ zu dieser pro Zeiteinheit gebildet wird. Die für jeden Inputstoff erwartbare Menge an Methan wurde für die Berechnungen dem KTBL-Heft 50, Gasausbeute in landwirtschaftlichen Biogasanlagen, Herausgeber Kuratorium für Technik und Bauwesen in der Landwirtschaft e. V., Darmstadt, 2005 entnommen. Der Mittelwert der Verteilung der relativen Substratausnutzung wurde durch Hölker (2013, persönliche Mitteilung) in einer umfangreichen Reihenuntersuchung ermittelt und liegt für eine Stichprobe von etwa 2.000 landwirtschaftlichen Biogasanlagen in Deutschland nahe 100%. Für die vorliegende Untersuchung wurde daher vereinfachend relativen Substratausnutzungen über 100% das Prädikat "gut" und relativen Substratausnutzungen unter 100% das Prädikat "schlecht" zugeordnet. Wie die Abbildungen 2, 4 und 6 zeigen, weichen die Mittelwerte der Aktivitäten von Cellulase, Xylanase und Gesamtesterasen in den Biomasse-Proben der so bewerteten beiden Gruppen deutlich voneinander ab und zwar derart, dass die Mittelwerte bei guter relativer Substratausnutzung höher sind, als bei schlechter. Dass dieser Zusammenhang vor dem Hintergrund der Verschiedenheit der Biogasanlagen innerhalb der Stichprobe sowie der zahlreichen sonstigen Einflüsse auf die relative Substratausnutzung so deutlich sichtbar wird, zeigt, dass die Aktivitäten der löslichen Fraktionen hydrolytischer Enzyme bzw. Enzymgruppen einen wesentlichen Faktor für die Gesamtleistung einer Biogasanlage darstellen und niedrige Aktivitäten relevanter Enzyme als ein Defizit zu deuten sind. Ein weiteres überraschendes Ergebnis ist, dass das Ausmaß der Korrelation der Enzymaktivitäten mit der relativen Substratausnutzung im Vergleich der Enzymgruppen untereinander verschieden ist, wenn man den relativen Abstand der Mittelwerte als Maß nimmt. So unterscheiden sich die Mittelwerte für Gruppen mit guter und schlechter relativer Substratausnutzung im Falle der Cellulaseaktivität um 16,2%, im Falle der Xylanaseaktivität um 11,5% und im Falle der Gesamtesteraseaktivität um 21,0%. Demnach ist für die untersuchte Stichprobe der Einfluss der Gesamtesterasen auf die relative Substratausnutzung von den untersuchten Enzymaktivitäten am höchsten. Nahezu keine Abhängigkeit findet man zwischen Relativer Substratausnutzung und der Lipaseaktivität (Abweichung der Mittelwerte 3,1%). Letzteres wird vor dem Hintergrund der Art der eingesetzten Inputstoffe in den landwirtschaftlichen Biogasanlagen, welche die Stichprobe bilden, und der Tatsache der im Vergleich zu Strukturpolysacchariden leichten Abbaubarkeit von Fetten verständlich. Im Gegensatz dazu zeigt der starke, zwischen der relativen Substratausnutzung und der Gesamtesteraseaktivität (gemessen mit dem Farbstoff Fluorescein-di-Acetat) gefundene Zusammenhang, die Bedeutung von Esterasen, welche kurzkettige Substituenten an Polysacchariden wie Acetat- und Methylreste (Acetylesterase, Methylesterase) abspalten und kovalente Brücken zwischen Polymeren (Feruloylesterase) spalten. Durch die Wirkung dieser Enzyme erst werden die Hauptketten der Polymere für hauptkettenspaltende Enzyme zugänglich.

### Bevorzugtes Auswahlkriterium für die Auswahl der enzymhaltigen Präparate gemäß der Ausgestaltung I:

Gemäß einer bevorzugten Ausführungsform der Ausgestaltung I des erfindungsgemäßen Verfahrens wird die tatsächliche Aktivität mindestens eines Enzyms oder einer Gruppe von Enzymen in der Biomasse im Bioreaktor mit einer geeigneten Methode ermittelt. Dazu wird die Biomasse gegebenenfalls vorbehandelt, zum Beispiel gemahlen, zerkleinert, gesiebt oder gemixt. Unterschreitet die Aktivität einen zugehörigen ermittelten Richtwert, so wird das betreffende Enzym oder die Enzymgruppe dem Biogasreaktor zugegeben. Dabei kann die Zugabe auf Fälle beschränkt werden, in denen eine deutliche Unterschreitung des Richtwertes (zum Beispiel um eine vorgegebene Toleranz) gegeben ist. Überschreitet die tatsächliche Aktivität des Enzyms oder der Enzymgruppe den Richtwert (ggfs. abzüglich der Toleranz), so kann von der Zugabe des Enzyms oder der Enzymklasse abgesehen werden. Bevorzugt wird für mehrere Enzyme oder Enzymgruppen die Einhaltung der betreffenden Richtwerte kontrolliert und gegebenenfalls durch Zugabe von Enzymen oder Gruppen von Enzymen sichergestellt. Die Enzymzugabe dient der Verbesserung der Hydrolyse und damit der Leistungssteigerung und Stabilisierung der Biogaserzeugung aus Biomasse. Wird ein Enzymmangel in der Biomasse ausgeglichen, so wird die Hydrolyserate erhöht und der Gesamtdurchsatz in der Biogasanlage gesteigert. Beispiel 1 erläutert die Ausgestaltung I des erfindungsgemäßen Verfahrens, ohne dass die Verfahrensausgestaltung auf dieses Beispiel beschränkt wäre. Dabei werden zum Ausgleich der ermittelten, fehlenden Enzymaktivitäten oder Aktivitäten von Gruppen von Enzymen, das oder diejenigen enzymhaltigen Präparate aus einer Gruppe vorhandener enzymhaltiger Präparate ausgewählt und gegebenenfalls so kombiniert, das für den Ausgleich des oder der Defizite rechnerisch die geringste Gesamtmenge an dem oder den enzymhaltigen Präparaten benötigt wird. Für diese Berechnung wird bevorzugt die Methode der Zielwertoptimierung verwendet. In einer weiteren Ausführungsform werden die spezifischen Kosten der enzymhaltigen Präparate mit bei der Auswahl dadurch mit berücksichtigt, dass die Zielwertoptimierung die spezifischen Kosten der enzymhaltigen Präparate als Parameter mit enthält.

### Ausgestaltung II des erfindungsgemäßen Verfahrens: Defizitmerkmal ist die Bildung von für die Hydrolyse maßgeblichen Enzymen auf eine induzierende Maßnahme hin:

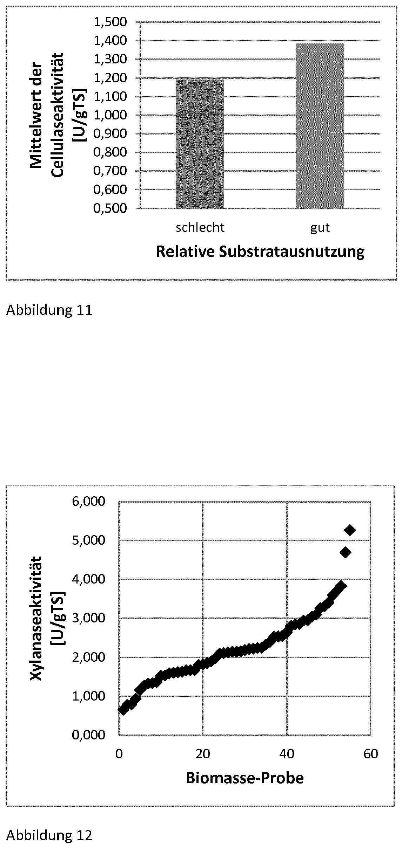

Gemäß einer weiteren bevorzugten Ausgestaltung II des erfindungsgemäßen Verfahrens wird die Fähigkeit zur Bildung von für die Hydrolyse maßgeblichen Enzymen oder Gruppen von Enzymen dadurch ermittelt, dass die zeitliche Zunahme der Enzymaktivitäten in der Biomasse aus einem Biogasreaktor nach einer enzyminduzierenden Maßnahme, beispielsweise der Zugabe eines oder mehrerer Inputstoffe in einer geeigneten Apparatur (beispielsweise einem temperierbaren Inkubationsgefäß mit der Möglichkeit, die Biomasse zur Rühren) nach einer vorgegebenen Inkubationszeit, relativ gegenüber einer Kontrolle ohne die erfolgte Maßnahme, also beispielsweise ohne Zusatz von Inputstoffen, gemessen wird. Überraschenderweise wurde nämlich beispielhaft gefunden, dass sich die relativen Zunahmen bestimmter Enzymaktivitäten in Biomasse-Proben aus verschiedenen Biogasanlagen stark voneinander unterscheiden und zudem von der Art des Inputstoffs abhängen. Dieser überraschende Effekt ist in den Abbildungen 9-13 in Anhang 3 dargestellt. Aus den Abbildungen 10-13 geht insbesondere hervor, dass die relative zeitliche Zunahme der Cellulaseaktivität bei Zugabe von Grassilage größer ist, als bei Zugabe von Maisssilage. Im Falle der Xylanaseaktivität ist es umgekehrt. Die relative zeitliche Zunahme bestimmter, für die Hydrolyse relevanter Enzymaktivitäten kann als Maß für die differenzielle Fähigkeit der Bioflora in der Biomasse eines Bioreaktors aufgefasst werden, auf einen bestimmten Fütterungsimpuls hin mit einer Steigerung der enzymatischen Hydrolyse durch die Bildung bestimmter Enzyme oder Gruppen von Enzymen zu reagieren.

### Bevorzugtes Auswahlkriterium für die Auswahl der enzymhaltigen Präparate zur Ausgestaltung II:

Gemäß einer bevorzugten Ausführungsform der Ausgestaltung II des erfindungsgemäßen Verfahrens wird die tatsächliche Zunahme der Aktivität mindestens eines Enzyms oder einer Gruppe von Enzymen in der Biomasse mit Hilfe der Apparatur ermittelt. Dazu wird die Biomasse gegebenenfalls vorbehandelt, zum Beispiel gemahlen, zerkleinert, gesiebt oder gemixt. Unterschreitet die Zunahme der Aktivität einen zugehörigen ermittelten Richtwert, so wird das betreffende Enzym oder die Enzymgruppe dem Biogasreaktor zugegeben. Dabei kann die Zugabe auf Fälle beschränkt werden, in denen eine deutliche Unterschreitung des Richtwertes (zum Beispiel um eine vorgegebene Toleranz) gegeben ist. Überschreitet die tatsächliche Zunahme der Aktivität des Enzyms oder der Enzymgruppe den Richtwert (ggfs. abzüglich der Toleranz), so kann von der Zugabe des Enzyms oder der Enzymklasse abgesehen werden. Bevorzugt wird für die Zunahme der Aktivität mehrerer Enzyme oder Enzymgruppen die Einhaltung der betreffenden Richtwerte kontrolliert und gegebenenfalls durch Zugabe von Enzymen oder Gruppen von Enzymen sichergestellt. Die Enzymzugabe dient der Verbesserung der Hydrolyse bei sich ändernden Betriebsbedingungen und damit der Leistungssteigerung und Stabilisierung der Biogaserzeugung aus Biomasse. Wird die mangelnde Fähigkeit zur Enzymbildung auf sich ändernde Bedingungen durch die Zugabe eines oder mehrerer enzymhaltiger Präparate in die Biomasse kompensiert, so wird die Hydrolyserate erhöht und der Gesamtdurchsatz in der Biogasanlage gesteigert. Dabei werden zum Ausgleich der ermittelten, fehlenden Enzymaktivitäten oder Aktivitäten von Gruppen von Enzymen, das oder diejenigen enzymhaltigen Präparate aus einer Gruppe vorhandener enzymhaltiger Präparate ausgewählt und gegebenenfalls so kombiniert, das für den Ausgleich des oder der Defizite rechnerisch die geringste Gesamtmenge an dem oder den enzymhaltigen Präparaten benötigt wird. Für diese Berechnung wird bevorzugt die Methode der Zielwertoptimierung verwendet. In einer weiteren Ausführungsform werden die spezifischen Kosten der enzymhaltigen Präparate mit bei der Auswahl dadurch mit berücksichtigt, dass die Zielwertoptimierung die spezifischen Kosten der enzymhaltigen Präparate als Parameter mit enthält.

### Ausgestaltung III des erfindungsgemäßen Verfahrens: Defizitmerkmal ist die Bildung von Biogas aus der Biomasse, gemessen in einer geeigneten Apparatur:

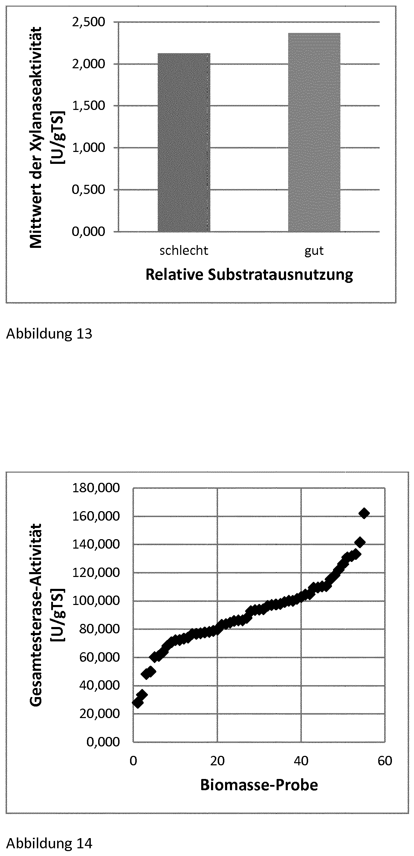

Gemäß einer weiteren Ausgestaltung III des erfindungsgemäßen Verfahrens besteht das quantitative Defizitmerkmal in der spezifischen Biogasbildung aus der Biomasse aus einem Biogasreaktor, gemessen in einer geeigneten Apparatur nach einer vorgegebenen Inkubationszeit. Dazu wird die Biomasse gegebenenfalls vorbehandelt, zum Beispiel gemahlen, zerkleinert, gesiebt oder gemixt. Die spezifische Biogasbildung aus Biomasse kann, bei vergleichsweise kurzen Inkubationszeiten, als Maß für die Qualität der Hydrolyse dienen. Eine hohe spezifische Biogasbildung innerhalb kurzer Inkubationszeiten deutet auf eine hohe Hydrolyserate hin, solange garantiert ist, dass dabei entstehende, energiereiche Metabolite sich nicht anhäufen. Bevorzugt wird die spezifische Biogasbildung mit Hilfe des ANKOM-Systems (für eine Beschreibung siehe zum Beispiel unter www.ankom.com) oder eines Eudiometers (für eine Beschreibung siehe zum Beispiel unter www.selutec.de) für Inkubationszeiten zwischen 24 und 140 Stunden ermittelt (siehe den Anhang 3). Bevorzugt beträgt die Inkubationstemperatur 40°C. Überraschenderweise hat sich nämlich gezeigt, dass die so ermittelte spezifische Biogasbildung aus Biomasse-Proben aus verschiedenen Biogasreaktoren nach einer Inkubationszeit von 140 Stunden erheblich variiert. Dies zeigt die Abbildung 14 in Anhang 3.

### Bevorzugte Ausführungsform der Ausgestaltung III:

Gemäß einer Ausführungsform der Ausgestaltung III wird der Biomasse vor oder während der Inkubation zur Messung der spezifischen Biogasbildung ein oder mehrere Inputstoffe oder andere Stoffe zugesetzt. Gegebenenfalls werden Inputstoffe oder Stoffe vorbehandelt, zum Beispiel gemahlen. Bevorzugt werden die Inputstoffe zum Beispiel aus der Gruppe der folgenden Stoffe ausgewählt: Maissilage, Grassilage, Getreide-Ganzpflanzensilage, Grünroggen, Rindermist, Pferdemist, Stroh, Wegebegleitgrün, Grünabfall, Hühnertrockenkot, Rindergülle und Schweinegülle. Bevorzugt werden die anderen Stoffe zum Beispiel aus der Gruppe der folgenden Stoffe ausgewählt: Carboxymethylcellulose, mikrokristalline Cellulose, Xylan, Arabinoxylan, Pektin, Rhamnogalacturonan, Xyloglucan und Lignin.

### Bevorzugtes Auswahlkriterium für die Auswahl der enzymhaltigen Präparate zur Ausgestaltung III:

Gemäß einer bevorzugten Ausführungsform der Ausgestaltung III des erfindungsgemäßen Verfahrens wird die tatsächliche spezifische Biogasmenge in einer Biomasse-Probe aus einem bestimmten Biogasreaktor in einer geeigneten Apparatur mit oder ohne Zugabe von gegebenenfalls vorbehandelten Inputstoffen oder anderen Stoffen vor oder während der Inkubation wie beschrieben ermittelt. Unterschreitet die gebildete Menge Biogas einen zugehörigen ermittelten Richtwert, so wird der Biomasse in dem Biogasreaktor mindestens ein enzymhaltiges Präparat in einer Menge oder solange zugegeben, bis der auf die gleiche Weise ermittelte spezifische Biogasmenge den Richtwert übersteigt. Dabei kann die Zugabe auf Fälle beschränkt werden, in denen eine deutliche Unterschreitung des Richtwertes (zum Beispiel um eine vorgegebene Toleranz) gegeben ist. Überschreitet die ermittelte spezifische Menge Biogas den Richtwert (ggfs. abzüglich der Toleranz), so kann von der Zugabe des einen oder der mehreren Enzympräparate abgesehen werden. Bevorzugt wird für die aus der Biomasse wie beschrieben gebildete spezifische Biogasmenge die Einhaltung der betreffenden Richtwerte kontrolliert und gegebenenfalls durch Zugabe von Enzymen oder Gruppen von Enzymen sichergestellt. Die Enzymzugabe dient der Verbesserung der Hydrolyse bezüglich der Substratausnutzung und damit der Leistungssteigerung und Stabilisierung der Biogaserzeugung aus Biomasse. Wird die Fähigkeit zur spezifischen Biogasbildung durch die Zugabe eines oder mehrerer enzymhaltiger Präparate in die Biomasse verbessert, so wird in dem Biogasreaktor die Hydrolyserate erhöht und der Gesamtdurchsatz in der Biogasanlage gesteigert.

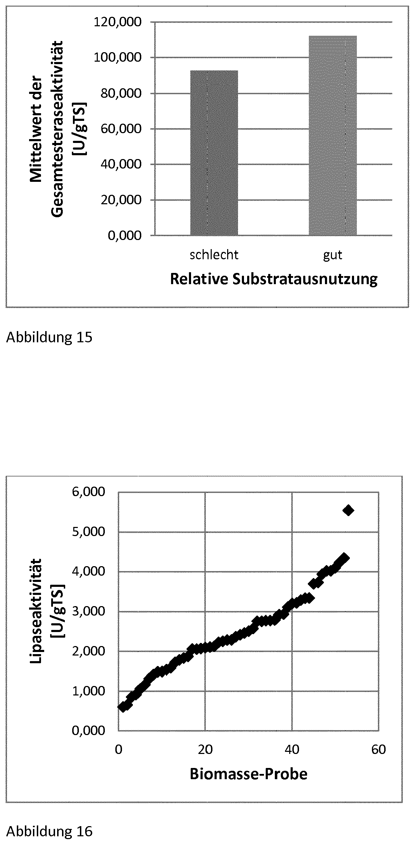

Dabei werden das oder die Enzympräparate so ausgewählt, dass aus einer gegebenen Gruppe von mindestens zwei enzymhaltigen Präparaten mindestens zwei Präparate einzeln und/oder in Kombination, wobei die Kombinationen unterschiedliche Anteile der mindestens zwei enzymhaltigen Präparate enthalten können, der Biomasse aus dem Biogasreaktor in einer geeigneten Apparatur zugesetzt werden, die spezifische Biogasbildung wie beschrieben ermittelt wird und das enzymhaltige Präparat oder die Kombination enzymhaltiger Präparate aus der gegebenen Gruppe enzymhaltiger Präparate der Biomasse in dem Biogasreaktor zugesetzt wird, welches oder welche, empirisch ermittelt, die höchste Menge an Biogas in dem durchgeführten Test ergibt. Überraschenderweise hat sich nämlich gezeigt, dass sich die Wirkung verschiedener enzymhaltiger Präparate auf die spezifische Biogasbildung aus einer Biomasse-Probe im Vergleich zur Kontrolle ohne Zusatz eines enzymhaltigen Präparats für verschiedene Biomasse-Proben aus verschiedenen Biogasreaktoren erheblich unterscheiden kann. Dies zeigt die Abbildung 15. In einer weiteren Ausführungsform werden die spezifischen Kosten der enzymhaltigen Präparate bei der Auswahl dadurch mit berücksichtigt, dass mit Hilf einer Zielwertoptimierung die enzymhaltigen Präparate ausgewählt werden, bei denen insgesamt das Verhältnis aus Wirkung und Kosten maximal ist.

### Ausgestaltung IV des erfindungsgemäßen Verfahrens: Defizitmerkmal sind die rheologischen Eigenschaften der Biomasse, gemessen in einer geeigneten Apperatur:

Gemäß einer weiteren Ausgestaltung IV des erfindungsgemäßen Verfahrens besteht das quantitative Defizitmerkmal in den rheologischen Eigenschaften der Biomasse aus einem Biogasreaktor, gemessen in geeigneten Apparaturen. Dazu wird die Biomasse gegebenenfalls vorbehandelt, zum Beispiel gemahlen, zerkleinert, gesiebt oder gemixt. Diese rheologischen Eigenschaften sollen hier durch den Begriff Zähigkeit bezeichnet werden, wobei es unterschiedliche Qualitäten von Zähigkeit gibt, die sich in unterschiedlichen Messverfahren verschieden stark ausprägen. Die Zähigkeit kann generell als ein Qualitätskriterium für den Biogasprozess und die Hydrolyse dienen, wobei eine geringe Zähigkeit vorteilhaft ist, da eine hohe Zähigkeiten eine hohe Rührenergie für die Homogenisierung der Biomasse und eine hohe Pumpenergie für die Förderung der Biomasse erfordert, den Verschleiß von Pumpen fördert und insgesamt hemmend auf den Biogasprozess wirkt. Dies alles hat wirtschaftliche Nachteile. Bevorzugt wird die Zähigkeit mit Hilfe eines Drehsmomentviskosimeters, eines Rotationsviskosimeters, eines Setzfließmaßes und/oder eines Auslaufviskosimeters gemessen. Dabei hat sich gezeigt, dass die Zähigkeit, gemessen mittels eines Drehmomentviskosimeters in Biomasse-Proben aus verschiedenen Biogasreaktoren erheblich variiert. Dies zeigt die Abbildung 16 in Anhang 3. Bevorzugt wird die Zähigkeit mit Hilfe des Drehmomentviskosimeters bei einer Umdrehung des Rührers von 200 rpm gemessen. Bevorzugt liegt die Messtemperatur bei 40°C.

### Bevorzugte Ausführungsform der Ausgestaltung IV:

Gemäß einer Ausführungsform der Ausgestaltung III wird der Biomasse vor oder während der Inkubation zur Messung der spezifischen Biogasbildung ein oder mehrere Inputstoffe oder andere Stoffe zugesetzt. Gegebenenfalls werden Inputstoffe oder Stoffe vorbehandelt, zum Beispiel gemahlen. Bevorzugt werden die Inputstoffe zum Beispiel aus der Gruppe der folgenden Stoffe ausgewählt: Maissilage, Grassilage, Getreide-Ganzpflanzensilage, Grünroggen, Rindermist, Pferdemist, Stroh, Wegebegleitgrün, Grünabfall, Hühnertrockenkot, Rindergülle und Schweinegülle. Bevorzugt werden die anderen Stoffe zum Beispiel aus der Gruppe der folgenden Stoffe ausgewählt: Carboxymethylcellulose, mikrokristalline Cellulose, Xylan, Arabinoxylan, Pektin, Rhamnogalacturonan, Xyloglucan und Lignin.

### Bevorzugtes Auswahlkriterium für die Auswahl der enzymhaltigen Präparate zur Ausgestaltung IV:

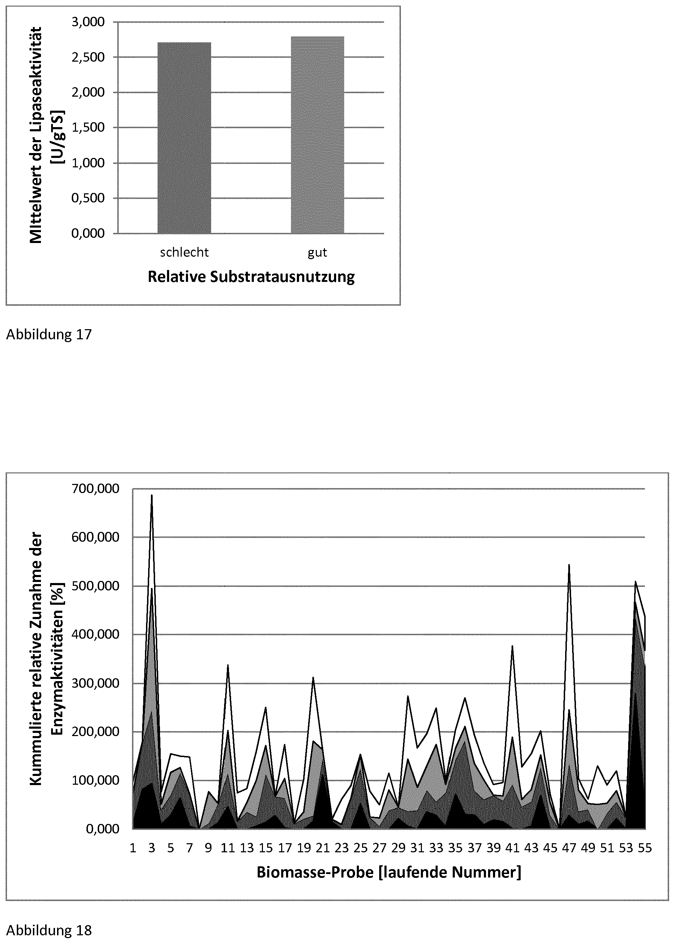

Gemäß einer bevorzugten Ausführungsform der Ausgestaltung IV des erfindungsgemäßen Verfahrens wird die tatsächliche Zähigkeit einer Biomasse-Probe aus einem bestimmten Biogasreaktor in einer geeigneten Apparatur mit oder ohne Zugabe von, gegebenenfalls vorbehandelten Inputstoffen oder anderen Stoffen vor oder während der Inkubation wie beschrieben ermittelt. Falls die in der Biomasse-Probe gemessene Zähigkeit oberhalb eines betreffenden Richtwertes liegt, wird der Biomasse in dem Biogasreaktor mindestens ein enzymhaltiges Präparat in einer Menge oder solange zugegeben, bis die auf die gleiche Weise ermittelte Zähigkeit den Richtwert unterschreitet. Dabei kann die Zugabe auf Fälle beschränkt werden, in denen eine deutliche Überschreitung des Richtwertes (zum Beispiel um eine vorgegebene Toleranz) gegeben ist. Unterschreitet die ermittelte Menge Biogas den Richtwert (ggfs. abzüglich der Toleranz), so kann von der Zugabe des einen oder der mehreren Enzympräparate abgesehen werden. Bevorzugt wird durch wiederholte Messung der Zähigkeit der Biomasse wie beschrieben die Einhaltung der betreffenden Richtwerte kontrolliert und gegebenenfalls durch Zugabe von Enzymen oder Gruppen von Enzymen sichergestellt. Die Enzymzugabe dient der Verminderung der Zähigkeit und damit der Verbesserung der Hydrolyse und damit wiederum einer Leistungssteigerung und Stabilisierung der Biogaserzeugung aus Biomasse. Wird durch die Enzymzugabe die Qualität der Hydrolyse erhöht, indem die Zähigkeit auf den Richtwert vermindert wird, so wird der Gesamtdurchsatz in der Biogasanlage gesteigert und der Aufwand für Rührenergie gesenkt. Dabei werden das oder die Enzympräparate so ausgewählt, dass aus einer gegebenen Gruppe von mindestens zwei enzymhaltigen Präparaten mindestens zwei Präparate einzeln und/oder in Kombination, wobei die Kombinationen unterschiedliche Anteile der mindestens zwei enzymhaltigen Präparate enthalten können, der Biomasse aus dem Biogasreaktor in einer geeigneten Apparatur zugesetzt werden und Änderung der Zähigkeit gegenüber einer Kontrolle ohne Zugabe eines enzymhaltigen Präparats nach einer gegebenen Inkubationszeit ermittelt wird. Bevorzugt liegt die Inkubationszeit zwischen 24 und 140 Stunden, besonders bevorzugt bei 140 Stunden. Es wird dasjenige enzymhaltige Präparat oder die Kombination enzymhaltiger Präparate aus der gegebenen Gruppe enzymhaltiger Präparate der Biomasse in dem Biogasreaktor zugesetzt, welches oder welche empirisch ermittelt die stärkste Absenkung der Zähigkeit gegenüber der Kontrolle ergibt. Überraschenderweise hat sich nämlich gezeigt, dass sich die Wirkung verschiedener enzymhaltiger Präparate auf die Absenkung der Zähigkeit, gemessen mit dem Drehmomentviskosimeter, in einer Biomasse-Probe im Vergleich zur Kontrolle ohne Zusatz eines enzymhaltigen Präparats für verschiedene Biomasse-Proben aus verschiedenen Biogasreaktoren erheblich unterscheiden kann. Dies zeigt die Abbildung 17. In einer weiteren Ausführungsform werden die spezifischen Kosten der enzymhaltigen Präparate bei der Auswahl dadurch mit berücksichtigt, dass mit Hilfe einer Zielwertoptimierung die enzymhaltigen Präparate ausgewählt werden, bei denen insgesamt das Verhältnis aus Wirkung und Kosten maximal ist.

### Weitere Ausgestaltungen, welche sich auf das gesamte Verfahren, unabhängig von den Ausgestaltungen I-IV beziehen:

Gemäß einer bevorzugten Ausgestaltung des erfindungsgemäßen Verfahrens wir ein Richtwert für ein Defizitmerkmal dadurch ermittelt, dass das Defizitmerkmal für eine Stichprobe von Biomasse-Proben aus Biogasreaktoren verschiedener Biogasanlagen ermittelt wird, die gemessenen Werte des Defizitmerkmals der Größe nach geordnet werden und als Richtwert derjenige Wert des Defizitmerkmals festgelegt wird, der ein vorgegebenes Größenkriterium erfüllt. Bevorzugt werden ja nach dem das 25%-Quantil oder das 75%-Quantil der empirischen Verteilung des Defizitmerkmals als Richtwert gewählt.

Gemäß einer weiteren bevorzugten Ausgestaltung des erfindungsgemäßen Verfahrens werden die Verteilungen der Defizitmerkmale und die Richtwerte für verschiedene Gruppen von Biogasanlagen aus einer Stichprobe getrennt ermittelt, wobei eine Gruppe von Biogasanlagen dadurch definiert ist, dass diese Biogasanlagen mindestens ein Merkmal gemeinsam haben. Bevorzugt werden das oder die Merkmale zum Beispiel aus der Gruppe folgender Merkmalen gewählt: Art, Menge und Qualität der Inputstoffe, Art der Vorbehandlung der Inputstoffe, Anzahl hintereinander geschalteter Biogasreaktoren, Raumbelastung des Fermenters, Verweilzeiten in den Biogasreaktoren, Art der verwendeten Rührwerke, Betriebstemperatur in den Biogasreaktoren, Einsatz prozessfördernder Präparate, stoffliche und biochemische Parameter des Gärschlamms (zum Gehalt an Trockensubstanz, Gehalt an organischer Trockensubstanz, Rohfasergehalt, NDF, ADF, ADL, Gehalt an Hemicellulosen, Gehalt an Lignin, pH-Wert, elektrische Leitfähigkeit, Ammoniumgehalt, Gehalt an anorganischem Puffer, Gehalt an Makroelementen, Gehalt an Spurenelementen, Gehalt an Metaboliten) und physikalische Parameter des Gärschlamm (rheologische Parameter, Partikelgrößenverteilung, Sedimentationsgrad, Entwässerungsvermögen).

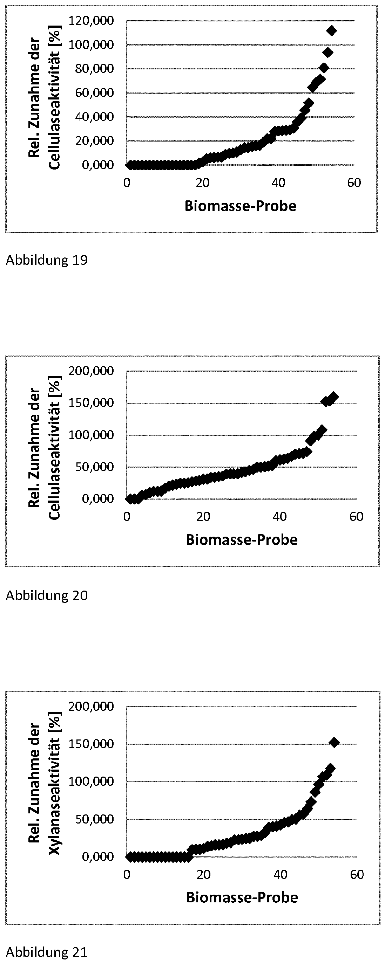

Gemäß einer anderen bevorzugten Ausgestaltung des erfindungsgemäßen Verfahrens werden die in einer Stichprobe von Biomasse-Proben aus Biogasreaktoren verschiedener Biogasanlagen gemessenen Werte für ein oder mehrere Defizitmerkmale mit den Aktivitäten von für die Hydrolyse relevanten Enzymen oder Gruppen von Enzymen korreliert und das Bestimmtheitsmaß der Korrelation ermittelt. Auf Basis der ermittelten Bestimmtheitsmaße werden dasjenige Enzym oder die Enzymgruppe für die Zugabe in die Biomasse eines Bioreaktors ausgewählt, für welche das ermittelte Bestimmheitsmaß oberhalb eines Schwellenwertes liegt. Beispielsweise wird der Biomasse aus dem Biogasreaktor in einer geeigneten Apparatur wie beschrieben Inputstoff zugesetzt, die Biogasbildung nach einer vorgegebenen Inkubationszeit gemessen und für die zu jedem Inputstoff gehörende Messreihe das Bestimmtheitsmaß der Regression der spezifischen Biogaserträge mit den in der Biomasse ermittelten Aktivitäten der Enzyme oder Enzymgruppen bestimmt. Auf Basis der ermittelten Bestimmtheitsmaße werden dasjenige Enzym oder die Enzymgruppe für die Zugabe in die Biomasse eines bestimmten Bioreaktors ausgewählt, für welche a) das ermittelte Bestimmheitsmaß oberhalb eines Schwellenwertes liegt. und b) die betreffende Biogasanlage, aus der die Biomasse entnommen wurde, überwiegend denjenigen Inputstoff einsetzt, für den ein Bestimmheitsmaß oberhalb des Schwellenwertes ermittelt wurde. ,Überwiegend' bedeutet hierbei, dass die Biogasanlage den Inputstoff in einem Anteil von bevorzugt mindestens 60% der Frischmasse einsetzt. Überraschenderweise hat sich nämlich gezeigt, dass die Korrelation der Gehalte verschiedener Enzyme oder Gruppen von Enzymen mit dem in einer Apparatur in einer gegebenen Inkubationszeit nach Zugabe von Inputstoff gebildeten spezifischen Menge Biogas von der Art des Inputstoffs abhängt, der der Biomasse zugesetzt wurde. Die Abbildungen 18 und 19 in Anhang 3 zeigen diesen Sachverhalt beispielhaft für die Cellulaseaktivität und die Inputstoffe Maissilage und Grassilage. In dem dargestellten Beispiel korreliert die in der Biomasse gemessene Cellulaseaktivität nennenswert nur mit dem spezifischen Biogasertrag, gemessen nach 48 h aus den Ansätzen, in denen Grassilage als Inputstoff zugesetzt wurde. Die spezifischen Biogaserträge nach 48 h Inkubationszeit sowohl mit Maissilage als auch mit Grassilage als Inputstoff korrelieren wiederum mit der Bewertung auf der Basis der relativen Substratausnutzung der Biogasanlagen, aus denen die Biomasseproben entnommen wurden (siehe die Abbildungen 20 und 21). Bevorzugt werden Maissilage oder Grassilage als die am häufigsten in der Praxis in landwirtschaftlichen Biogasanlagen eingesetzten Inputstoffe verwendet und der Biogasertrag bevorzugt mit Hilfe des ANKOM-Verfahrens ermittelt. Weiterhin bevorzugt werden die Bestimmheitsmaße auf Basis einer linearen Regression ermittelt.

Gemäß einer anderen bevorzugten Ausgestaltung des erfindungsgemäßen Verfahrens werden die Richtwerte periodisch überprüft und die Zugabe der entsprechend ausgewählten enzymhaltigen Präparate entsprechend angepasst. Bevorzugt werden die Richtwerte zu Beginn der Enzymzugabe einmal wöchentlich, später einmal monatlich überprüft.

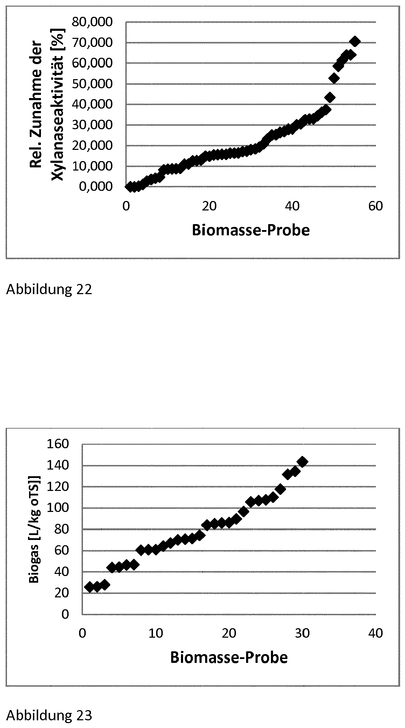

Gemäß einer bevorzugten Ausgestaltung des erfindungsgemäßen Verfahrens werden das oder die Defizitmerkmale, auf Basis derer in Verbindung mit den zugehörigen Richtwerten und Auswahlkriterien das oder die enzymhaltigen Präparate zur Verbesserung der Hydrolyse ermittelt werden, anhand bestimmter Regeln ausgewählt. Wie die Abbildung 22 zeigt, korreliert die gemäß der Ausgestaltung III des erfindungsgemäßen Verfahrens gemessene spezifische Biogasbildung aus Biomasse-Proben aus Bioreaktoren nach 140 Stunden Inkubationszeit linear mit der mit Hilfe des Drehmomentviskosimeters in den gleichen Proben gemessenen Anfangszähigkeit. Dabei ist die Anfangszähigkeit als ursächlich gegenüber der spezifischen Biogasbildung anzusehen. In die spezifische Biogasbildung fließen aber außer der Viskosität zahlreiche weitere Faktoren ursächlich mit ein, beispielsweise die Freisetzung energiereicher Metabolite im Zuge der Hydrolyse. Weitere Korrelationen sind in den Abbildungen 2, 4, 6 und 19 gezeigt. Die Abweichungen der im Rahmen der Ausgestaltungen I-IV des erfindungsgemäßen Verfahrens spezifizierten Defizitmerkmale von Ihren Richtwerten beleuchten demnach verschiedene, wenn auch mit einander zusammenhängende Aspekte der Qualität der Hydrolyse in der Biomasse aus einem Biogasreaktor. Nach einer bevorzugten Ausführungsform der vorliegenden Ausgestaltung des erfindungsgemäßen Verfahrens werden zwei Defizitmerkmale in folgender Reihenfolge nacheinander geprüft und ggfs. durch das oder die gemäß der jeweiligen Ausgestaltungen des erfindungsgemäßen Verfahrens ausgewählten enzymhaltigen Präparate beseitigt: Zähigkeit (Ausgestaltung IV) - > Spezifische Biogasbildung (Ausgestaltung III). Dieser Reihenfolge liegt die folgende Logik zugrunde: Die Beseitigung einer Abweichung der Zähigkeit der Biomasse in einem Bioreaktor von dem bevorzugten Richtwert fördert die spezifische Biogasbildung (siehe Abbildung 22) und vermindert den Rühraufwand. Beides trägt zur Wirtschaftlichkeit des Biogasprozesses bei. Falls anschließend eine Abweichung der spezifischen Biogasbildung noch bestehen sollte, so kann diese mit einem geringeren Einsatz eines ggfs. anderen, passenden enzymhaltigen Präparats, welches gemäß der Ausgestaltung (Ausgestaltung III) ausgewählt wird, beseitigt werden. In einer anderen, bevorzugten Ausführungsform der vorliegenden Ausgestaltung werden zunächst die Defizite in den Aktivitäten der für die Hydrolyse relevanten Enzyme oder Gruppen von Enzymen an die jeweiligen Richtwerte angepasst. Falls anschließend Abweichungen der Zähigkeit oder der spezifischen Biogasbildung von den jeweiligen Richtwerten noch bestehen, werden diese gemäß der in den Ausgestaltungen III und IV beschriebenen Ausführungen beseitigt. Dabei wird zuerst die Abweichung der Zähigkeit von dem betreffenden Richtwert beseitigt. Für den Fall, dass die Biomasse aus einem Biogasreaktor eine gemessene Zähigkeit hat, die unterhalb des betreffenden, bevorzugten Richtwertes liegt, wird bevorzugt die Abweichung der spezifischen Biogasbildung von dem betreffenden Richtwert geprüft und gegebenenfalls durch die Zugabe eines oder mehrerer enzymhaltiger Präparate, welche gemäß den Ausführungen der Ausgestaltung III ausgewählt wurden, beseitigt. Falls die Betriebsführung einer Biogasanlage auf die Zufuhr anderer Inputstoffe geändert werden soll, wird bevorzugt die Fähigkeit zur Enzymbildung gemäß der Ausgestaltung II überprüft.

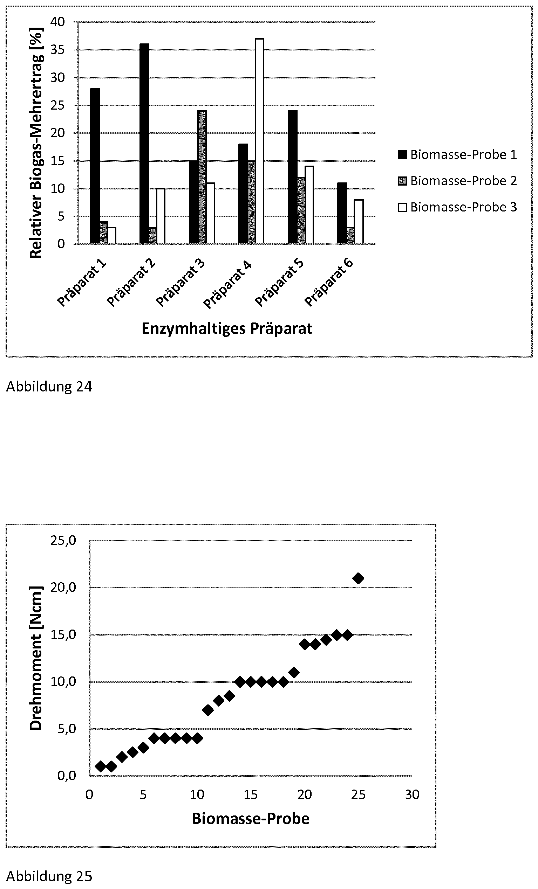

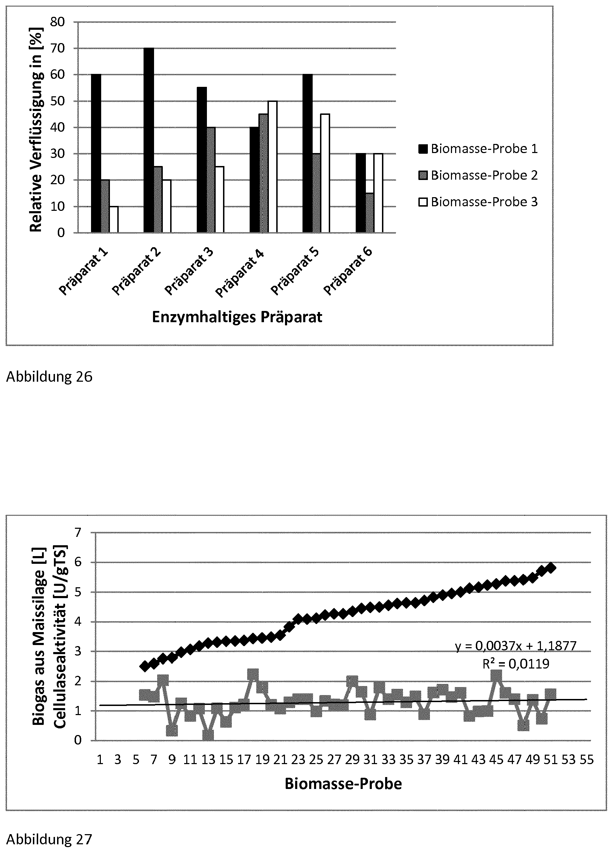

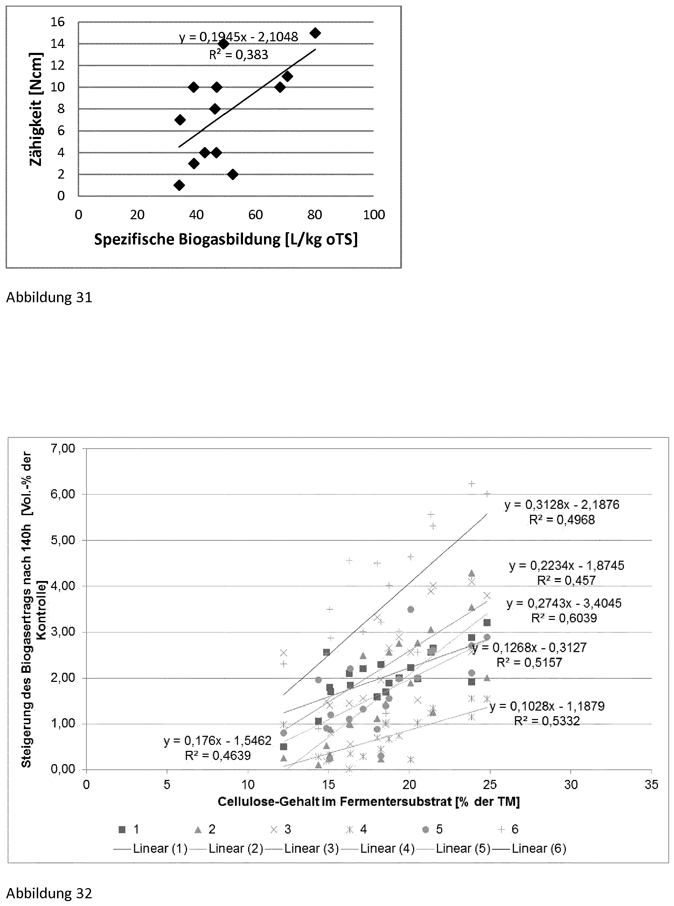

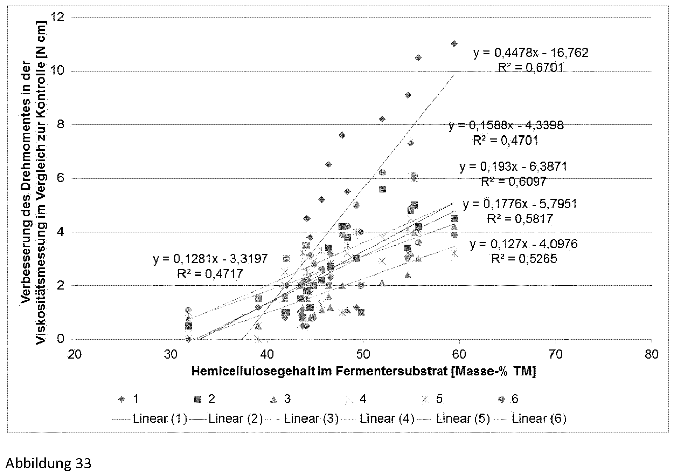

Gemäß einer weiteren bevorzugten Ausgestaltung erfindungsgemäßen Verfahrens werden das oder die enzymhaltigen Präparate anhand mindestens eines Merkmals der Biomasse aus einem Biogasreaktor ausgewählt, welches mit einem oder mehreren Defizitmerkmalen aus der Biomasse korreliert. Überraschenderweise hat sich nämlich gezeigt, dass die Wirkung verschiedener enzymhaltiger Präparate oder Kombinationen enzymhaltiger Präparate auf die spezifische Biogasbildung aus Biomasse-Proben aus verschiedenen Biogasreaktoren mit bestimmten anderen Merkmalen der Biomasse korreliert. Beispielsweise zeigt die Abbildung 23 in Anhang 3, dass die Wirkung verschiedener getesteter enzymhaltiger Präparate auf die spezifische Biogasbildung aus Biomasse-Proben mit dem Cellulose-Gehalt der Biomasse-Proben korreliert. Deshalb kann beispielsweise der Cellulose-Gehalt der Biomasse in einem Biogasreaktor mit Hilfe einer geeigneten Methode bestimmt und im Falle eines hohen gemessenen Hemicellulose-Gehalts der Biomasse, das oder die enzymhaltigen Präparate oder Kombinationen der Präparate der Biomasse in dem Biogasreaktor zur Verbesserung der spezifischen Biogasbildung aus der Biomasse zugesetzt werden, bei denen das Bestimmtheitsmaß der Korrelation der spezifischen Biogasbildung aus der Biomasse mit dem Cellulose-Gehalt der Biomasse einen bestimmten Schwellwert überschreitet oder maximal ist. Dabei kann die Korrelation positiv oder negativ sein. Bevorzugt werden diese Merkmale der Biomasse aus der Gruppe der folgende Merkmale ausgewählt: Trockenmassegehalt, Gehalt an organischer Trockenmasse, Rohfaser-Gehalt, NDF-Gehalt, ADF-Gehalt, ADL-Gehalt, Gehalt an Hemicellulosen, Gehalt an Cellulose, Gehalt an Pektinen, Gehalt an Lignin, Gehalt an Makro- und Spurenelementen, pH-Wert, elektrische Leitfähigkeit, Aktivitäten von Enzymen. Auf diese Weise kann die Messung der spezifischen Biogasbildung aus einer Biomasseprobe durch eine möglicherweise einfachere Messung eines anderen Merkmals dann ersetzt werden, wenn die Wirkung bestimmter enzymhaltiger Präparate auf die spezifische Biogasbildung mit der Ausprägung dieses anderen Merkmals korreliert. Weiterhin bevorzugt werden die ermittelten Bestimmtheitsmaße für mehrere Merkmale miteinander verglichen und in Verbindung gebracht. Daraus können logische Regeln entwickelt werden, die festlegen, unter welchen Bedingungen ein oder mehrere enzymhaltige Präparate ausgewählt werden. Beispielsweise könnte daraus abgeleitet werden, dass ein bestimmtes enzymhaltiges Präparat dann ausgewählt wird, wenn zwei unterschiedliche Merkmale A und B zugleich in hoher Ausprägung in der Biomasse aus dem Bioreaktor vorliegen, ein anderes Präparat, wenn Merkmal A in hoher und Merkmal B in niedriger Ausprägung vorliegt. Die Abbildung 24 in Anhang 3 zeigt ein weiteres Beispiel einer solchen Korrelation zwischen der Wirkung verschiedener enzymhaltiger Präparate auf die Verbesserung der Zähigkeit der Biomasse, gemessen mit einem Drehmomentviskosimeter, von dem Hemicellulose-Gehalt der Biomasse.

Gemäß einer anderen bevorzugten Ausgestaltung des erfindungsgemäßen Verfahrens werden die Enzyme aus der Liste folgender Enzyme, aus denen sich die enzymhaltigen Präparate zusammensetzen, bevorzugt aus folgender Gruppe von Enzymen ausgewählt: Cellobiohydrolase, beta-1,4-Endoglucanase, beta-1,4-Glucosidase, beta-1,4-Endoxylanase, beta-1,4-Xylosidase, beta-1,4(1,3)-Endoglucanase, Xyloglucan-beta-1,4-Endoglucanase, Exopolygalacturonase, Endopolygalacturonase, Pektinlyase, Pektatlyase, Rhamnogalacturonanlyase, beta-1,4-Endomannanase, beta-1,4-Endogalactanase, Mannosidase, Exoarabinase, Endoarabinase, Lichenase, Laminarinase, alpha-1,4-Galactosidase, beta-1,4-Galactosidase, alpha-Arabinofuranosidase, alpha-Xylosidase, alpha-Fucosidase, alpha-Glucuronidase, alpha-Rhamnosidase, Feruloylesterase, Acetylesterase, Methylesterase. Dabei haben die Untersuchungen gezeigt, dass besonders Enzyme, die lange und kurze Seitenketten der Polymere abspalten, über Ester substituierte kleine Liganden wie Acetyl- und Methylreste entfernen und kovalente Quervernetzungen (Feruloyl-di-Ester) zwischen den Makromolekülen spalten, besonders bedeutsam sind. Bevorzugt werden deshalb Enzyme aus der folgenden Gruppe ausgewählt und der Biomasse in dem Biogasreaktor zugesetzt: Feruloylesterase, Acetylesterase, Methylesterase.

Gemäß einer bevorzugten Ausgestaltung des erfindungsgemäßen Verfahrens wird mindestens ein Metabolit, bezogen auf die Biomasse aus einem Biogasreaktor, der als Indikator für eine Limitierung durch die Hydrolyse dient, und mindestens ein Messverfahren für die Messung der Konzentration des mindestens einen Metaboliten in der Biomasse angegeben. Des Weiteren wird mindestens ein Richtwert für die Konzentration des mindestens einen Metaboliten in der Biomasse zur Verfügung gestellt. Gemäß dieser bevorzugten Ausgestaltung erfolgt eine Zugabe des einen oder der mehreren enzymhaltigen Präparate zu der Biomasse in einem Biogasreaktor nur, solange die in der Biomasse gemessene Konzentration des mindestens einen Metaboliten unterhalb des mindestens einen Richtwertes ist. Bevorzugt wird der Metabolit aus der Gruppe der folgenden Metabolite gewählt: Essigsäure, Propionsäure, Buttersäure, iso-Buttersäure. Die Konzentration des Metaboliten in der Biomasse kann mit Hilfe verschiedener Messmethoden ermittelt werden. Bevorzugt wird die Konzentration des Metaboliten in der Biomasse mit Hilfe des Verfahrens der Gaschromatografie ermittelt. Bevorzugte Richtwerte für die bevorzugten Metabolite sind: flüchtige organische Fettsäuren, sonstige organische Säuren, Zucker und Zuckerderivate, Aminosäuren und Alkohole. Besonders bevorzugt als Metabolit ist Essigsäure. Untersuchungen weitere Erkenntnisse gewonnen werden können, welche zu geeigneteren Metaboliten, besseren Messmethoden und genaueren Richtwerten führen.

Gemäß einer bevorzugten Ausgestaltung des erfindungsgemäßen Verfahrens werden das oder die Enzympräparate direkt in die Biomasse in dem Biogasreaktor gegeben.

Gemäß einer weiteren bevorzugten Ausgestaltung des erfindungsgemäßen Verfahrens werden das oder die Enzympräparate täglich in einer gleichen Menge in die Biomasse gegeben. In einer anderen bevorzugten Ausgestaltung des erfindungsgemäßen Verfahrens werden das oder die enzymhaltigen Präparate in einer täglich variierenden Menge zugegeben. Bevorzugt werden das oder die enzymhaltigen Präparate zu Beginn der Enzymzugabe in höherer Menge und in der Folge abnehmender Menge der Biomasse in dem Bioreaktor zugegeben. Weiterhin bevorzugt werden das oder die enzymhaltigen Präparate in einer zeitlich oszillierenden Dosierung oder sonst einem definierten zeitlichen Dosierungsprofil der Biomasse zugegeben.

Gemäß einer Ausgestaltung des erfindungsgemäßen Verfahrens werden im Falle mehrerer ausgewählter enzymhaltiger Präparate diese gleichzeitig aber getrennt voneinander in die Biomasse in dem Bioreaktor gegeben. Gemäß einer weiteren Ausgestaltung werden mehrere ausgewählte enzymhaltige Präparate vor der Zugabe in den Biogasreaktor gemischt. Gemäß einer anderen Ausgestaltung werden die ausgewählten enzymhaltigen Präparate zu verschiedenen Zeiten in die Biomasse in dem Biogasreaktor gegeben. Bevorzugt wird hierfür ein definiertes zeitliches Dosierungsschema vorgegeben. Besonders bevorzugt orientiert sich dieses Dosierungsschema an Merkmalen der Betriebsführung der Biogasanlage, beispielsweise an der Fütterung der Biogasanlage mit Inputstoffen und/oder an Rührereignissen und/oder der Zugabe anderer prozessfördernder Hilfsmittel.

In einer anderen bevorzugten Ausgestaltung des erfindungsgemäßen Verfahrens werden das oder die Enzympräparate gemeinsam mit anderen Prozesshilfsmitteln, bevorzugt mit spurenelementehaltigen Präparaten der Biomasse in dem Bioreaktor zugegeben. Durch die Beschleunigung der Hydrolase kann einer der nachfolgenden Prozessschritte des Biogasprozesses unbeabsichtigt limitierend wirken, insbesondere die Methanogenese. Durch die gleichzeitige Zugabe spurenelementehaltiger Präparate kann dieser Effekt verhindert werden.

Gemäß einer weiteren bevorzugten Ausgestaltung des erfindungsgemäßen Verfahrens können die enzymhaltigen Präparate in fester oder flüssiger Form in die Biomasse in dem Biogasreaktor gegeben werden. Bevorzugt werden die Präparate fester Form zugegeben.

Gemäß einer weiteren bevorzugten Ausgestaltung des erfindungsgemäßen Verfahrens werden das oder die enzymhaltigen Präparate zusammen mit Wirkstoffen der Biomasse zugegeben, welche die katalytische Wirkung und die Bioverfügbarkeit der Enzyme fördern. Bevorzugt werden die Wirkstoffe zum Beispiel aus der Gruppe folgender Wirkstoffe ausgewählt: spezifische, für die katalytische Wirkung bestimmter Enzyme notwendige Metalle, oberflächenaktive Stoffe und Chelatbildner, durch welche inhibierende Ionen komplexiert werden.

Gemäß einer weiteren bevorzugten Ausgestaltung des erfindungsgemäßen Verfahrens werden das oder die enzymhaltigen Präparate zusammen mit Wasser der Biomasse so zugegeben, dass die Wasseraktivität in der Biomasse einen vorgegebenen Schwellenwert übersteigt. Hierdurch wird die Beweglichkeit der Enzymmoleküle (Diffusionskoeffizient) erhöht und die katalytische Wirkung verbessert.

Gemäß einer anderen bevorzugten Ausgestaltung des erfindungsgemäßen Verfahrens werden die Enzyme dadurch vor Inaktivierung durch Wärme, Proteasen oder sonstige Einflüsse seitens der Zusammensetzung und der physikalischen Eigenschaften der Biomasse geschützt, dass sie in einer geeigneten Weise formuliert werden, beispielweise mit Hilfe eines Coating-Verfahrens gekapselt werden. In einer besonderen Ausführung dieser Ausgestaltung dient das Coating der Verlangsamung der Freisetzung der Enzyme aus den Bestandteilen des enzymhaltigen Präparats in die Biomasse.

### Beispiel zur Ausgestaltung I des erfindungsgemäßen Verfahrens

Aus den Größenverteilungen der in einer Stichprobe von Biomasse-Proben aus verschiedenen Biogasreaktoren für die Aktivitäten von Cellulase, Xylanase, Polygalacturonase und Gesamtesterasen gemessenen Aktivitäten, lassen sich folgende Richtwerte ableiten und zur Verfügung stellen:

| Cellulase | Xylanase | Polygalacturonase | Gesamtesterasen |
|---|---|---|---|
| 1,7 U/gTS | 3,1 U/gTS | 3,5 U/gTS | 110,6 U/gTS |

Für 4 Biomasse-Proben aus der Stichprobenmenge wurden die Aktivitäten von Cellulase, Xylanase, Polygalacturonase und Gesamtesterasen nach folgenden Protokollen folgt ermittelt:

### Probenvorbereitung:

Die Biomasse-Probe aus dem Bioreaktor wird bei -80°C eingefroren. Vor der Durchführung der Messung wird die Biomasse-Probe über Nacht im Kühlschrank auftaut. Je 10g der Probe werden in Falcon-Tubes eingewogen, 40 ml Aqua. Dest. zugegeben (1:5 Verdünnung) und die Suspension 5 min in einem Vortex-Mischer homogenisiert. Anschließend wird der Inhalt auf 2-mL-Reaktionsgefäße verteilt und bei 13.000 rpm 10 min zentrifugiert. Der Überstände (Enzymlösungen) werden in ein neues Reaktionsgefäß überführt und bis zur Messung auf Eis kühl gehalten.

### Messung der Cellulaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 150 µL Enzymlösung 350 µl Substrat (1% Carboxymethylcellulose in 250 mM Kaliumphosphatpuffer, pH 7.0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Glucose-Standards erstellt wird.

### Messung der Xylanaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 150 µL Enzymlösung 350 µl Substrat (1,5% Xylan in 250mM Kaliumphosphatpuffer, pH 7,0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Xylan-Standards erstellt wird.

### Messung der Polygalacturonaseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 100 µL Enzymlösung 200 µl Substrat (1,2% Polygalacturonsäure in 250mM Kaliumphosphatpuffer, pH 7,0) gegeben und der Ansatz 30 min bei 40°C auf einem Thermoschüttler inkubiert. Nach der Inkubation werden 750 µl Dinitrosalicylsäurereagenz zugegeben, die Lösung 10 min gekocht und anschließend 5 min auf Eis gekühlt. Die abgekühlte Lösung wird 5 min wie oben zentrifugiert. Anschließend erfolgt die Messung der Lichtabsorption bei 575 nm. Die Berechnung der Aktivität erfolgt mit Hilfe einer Kalibrationskurve, welche mithilfe eines Galacturonsäure-Standards erstellt wird.

### Messung der Gesamtesteraseaktivität:

Die Messung wird 3-fach parallel gegenüber einer Kontrolle durchgeführt, bei der die Enzymlösung durch Autoklavieren bei 120°C für 30 min inaktiviert wurde. Für die Messung werden zu 100 µL Enzymlösung 880 µl Phosphat-Puffert (200 mM, pH 7,0) und 20 µL Fluorescein-di-Acetat (2 mg/mL in Aceton) gegeben und der Ansatz 20 min bei 40°C auf einem Thermoschüttler inkubiert. Nach der Inkubation wird 1 mL Aceton (50%ig) zugegeben und 5 min wie oben zentrifugiert. Anschließend erfolgt die Messung der Lichtabsorption bei 490 nm. Die Berechnung der Aktivität erfolgt mit Hilfe einer Kalibrationskurve, welche mithilfe eines Fluorescein-di-Acetat-Standards erstellt wird.

In der Biomasse aus dem beprobten Biogasreaktor wurden folgende Enzymaktivitäten gemessen:

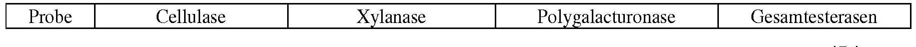

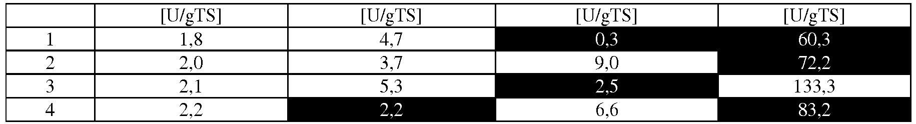

Dabei liegen bei den schwarz unterlegten Feldern die gemessenen Aktivitäten unterhalb der Richtwerte. Nach diesen Analyseergebnissen wurden den zu den jeweiligen Proben zugeordneten Biogasreaktoren folgende Enzyme zugegeben:
- Probe 1:: Polygalacturonase, Acetylesterase, Methylesterase, Feruloylesterase, Arabinofuranosidase.
- Probe 2:: Acetylesterase, Methylesterase, Feruloylesterase, Arabinofuranosidase.
- Probe 3:: Polygalacturonase
- Probe 4:: Xylanase, Acetylesterase, Methylesterase, Feruloylesterase, Arabinofuranosidase.

## Patentansprüche

1. Verfahren zur Erzeugung von Biogas in einem Biogasreaktor umfassend die folgenden Schritte:
- mindestens ein Merkmal, bezogen auf die Biomasse aus einem Biogasreaktor, wird angegeben,
- mindestens ein Messverfahren für die Messung des mindestens einen Merkmals wird angegeben,
- mindestens ein Richtwert für das mindestens eine Merkmal wird zur Verfügung gestellt,
- in dem Biogasreaktor wird Biogas erzeugt,
- der Wert des mindestens einen Merkmals in der Biomasse aus dem Biogasreaktor wird mit Hilfe des mindestens einen Messverfahrens ermittelt,
- mindestens ein Auswahlkriterium für die Auswahl mindestens eines enzymhaltigen Präparates aus einer gegebenen Gruppe von mindestens zwei enzymhaltigen Präparaten wird zur Verfügung gestellt,
- falls der ermittelte Merkmalswert in der Biomasse von dem mindestens einen zur Verfügung gestellten Richtwert abweicht, werden das oder die gemäß des mindestens einen Auswahlkriteriums ausgewählten enzymhaltigen Präparate in einer solchen Menge oder über einen solchen Zeitraum der Biomasse in den Biogasreaktor zugegeben, bis die gemessene Abweichung verschwindet,
- bei dem das Merkmal die Aktivität mindestens eines für die Hydrolyse maßgeblichen Enzyms oder einer maßgeblichen Gruppe von Enzymen in der Biomasse ist und bei dem dasjenige Enzym oder diejenige Enzymgruppe der Biomasse in dem Biogasreaktor zugegeben wird, für das oder für die die ermittelte Aktivität unterhalb des zur Verfügung gestellten Richtwertes liegt.

2. Verfahren nach Anspruch 1, wobei der Richtwert aus der empirischen Häufigkeitsverteilung der Aktivität des mindestens einen Enzyms in einer Stichprobe von Biomasseproben aus Biogasreaktoren ermittelt wird.

3. Verfahren nach Anspruch 2, wobei der Richtwert das 75%-Quantil der empirischen Häufigkeitsverteilung ist.

4. Verfahren nach einem der Ansprüche 1 bis 3, wobei der Richtwert empirisch aus der Wirkung von mindestens zwei enzymhaltigen Präparaten auf den Abbau von Inputstoffen oder Inputstoffgemischen abgeleitet wird.

5. Verfahren nach einem der Ansprüche 1 bis 3, wobei der Richtwert empirisch aus der stofflichen Zusammensetzung der Inputstoffe oder der Biomasse in einem Bioreaktor abgeleitet wird.

6. Verfahren nach einem der Ansprüche 1 bis 5, wobei für die Zugabe der ermittelten Enzyme oder Gruppen von Enzymen aus einer Gruppe von mindestens zwei enzymhaltigen Präparaten mit verschiedenen Enzymaktivitäten dasjenige Präparat oder diejenigen Präparate in einer geeigneten Kombination so ausgewählt werden, dass die ermittelten Abweichungen der Enzymaktivitäten von ihren Richtwerten mit der geringsten Gesamtmenge oder den geringsten Gesamtkosten des oder der enzymhaltigen Präparate beseitigt werden können und das hierfür die Methode der Zielwertoptimierung angewendet wird.

7. Verfahren nach einem der Ansprüche 1 bis 6, bei dem das Merkmal die in der Biomasse aus einem Biogasreaktor gemessene Zähigkeit ist und bei dem aus einer gegebenen Gruppe von mindestens zwei enzymhaltigen Präparaten mindestens zwei Präparate einzeln und/oder in Kombination der Biomasse aus dem Biogasreaktor zugesetzt werden, die zeitliche Änderung der Zähigkeit gegenüber einer Kontrolle ohne Zugabe eines enzymhaltigen Präparats ermittelt wird und dasjenige enzymhaltige Präparat oder diejenige Kombination enzymhaltiger Präparate aus der gegebenen Gruppe enzymhaltiger Präparate der Biomasse in dem Biogasreaktor zugesetzt wird, welches oder welche empirisch ermittelt die stärkste Absenkung der Zähigkeit gegenüber der Kontrolle ergibt.

8. Verfahren nach einem der Ansprüche 1 bis 7, bei dem das Merkmal die spezifische Biogasbildung aus der Biomasse aus einem Biogasreaktor ist und bei dem aus einer gegebenen Gruppe von mindestens zwei enzymhaltigen Präparaten mindestens zwei Präparate einzeln und/oder in Kombination der Biomasse aus dem Biogasreaktor zugesetzt werden, die spezifische Bildung von Biogas aus der Biomasse gegenüber einer Kontrolle ohne Zugabe eines enzymhaltigen Präparats ermittelt wird und dasjenige enzymhaltige Präparat oder diejenige Kombination enzymhaltiger Präparate aus der gegebenen Gruppe enzymhaltiger Präparate der Biomasse in dem Biogasreaktor zugesetzt wird, welches oder welche die höchste spezifische Biogasbildung gegenüber der Kontrolle ergibt.

9. Verfahren nach einem der Ansprüche 1 bis 8, wonach der Richtwert aus der empirischen Häufigkeitsverteilung des Merkmals in einer Stichprobe von Biomasseproben aus Biogasreaktoren ermittelt wird.

10. Verfahren nach Anspruch 9, wobei der Richtwert ein Quantil der empirischen Häufigkeitsverteilung ist.

11. Verfahren nach einem der Ansprüche 1 bis 10, wonach Richtwerte für verschiedene Gruppen von Biogasanlagen aus einer Stichprobe getrennt ermittelt werden und eine Gruppe von Biogasanlagen dadurch definiert ist, dass diese Biogasanlagen mindestens eines der folgenden Merkmale gemeinsam haben: einen gemeinsamen Inputstoff, eine gemeinsame Art der Vorbehandlung der Inputstoff, eine gemeinsame Anzahl hintereinandergeschalteter Biogasreaktoren, eine gemeinsame Raumbelastung des Fermenters, gemeinsame Verweilzeiten in den Biogasreaktoren, eine gemeinsame Art der verwendeten Rührwerke, gemeinsame Betriebstemperaturen im Biogasreaktor, ein gemeinsamer Einsatz prozessfördernder Hilfsmittel und/oder gemeinsame physikalische oder biochemische Parameter des Gärschlamms und ermittelt wird, in welche Gruppe von Biogasanlagen ein Biogasreaktor fällt und für das Verfahren zur Erzeugung von Biogas in dem Biogasreaktor die Richtwerte der betreffenden Gruppe von Biogasanlagen herangezogen werden.

12. Verfahren nach einem der Ansprüche 7 bis 11, bei dem das Merkmal Zähigkeit bevorzugt vor den anderen Merkmalen geprüft und eine eventuelle Abweichung durch eine Zugabe eines oder mehrerer enzymhaltiger Präparate beseitigt wird.

13. Verfahren nach einem der Ansprüche 1 bis 12, bei dem das Merkmal Enzymaktivitäten bevorzugt vor den anderen Merkmalen geprüft und eine eventuelle Abweichung durch eine Zugabe eines oder mehrerer enzymhaltiger Präparate beseitigt wird.

14. Verfahren nach einem der Ansprüche 1 bis 13, bei dem mindestens ein Metabolit oder ein Parameter, bezogen auf die Biomasse aus einem Biogasreaktor, der als Indikator für eine Limitierung dient und mindestens ein Messverfahren für die Messung der Konzentration des mindestens einen Metaboliten oder des Werts des mindestens einen Parameters in der Biomasse angegeben wird, ein Richtwert für die Konzentration des mindestens einen Metaboliten oder den mindestens einen Parameter in der Biomasse zur Verfügung gestellt wird und die Zugabe eines oder mehrerer enzymhaltiger Präparate zu der Biomasse in dem bestimmten Biogasreaktor nur erfolgt, solange die in der Biomasse gemessene Konzentration des mindestens einen Metaboliten oder der Wert des mindestens einen Parameters unterhalb oder oberhalb des Richtwerts liegt.

## Claims

1. A method for generating biogas in a biogas reactor, comprising the following steps:
- at least one feature is indicated with reference to the biomass from a biogas reactor,
- at least one measuring method for measuring the at least one feature is indicated,
- at least one reference value for the at least one feature is provided,
- biogas is generated in the biogas reactor,
- the value of the at least one feature in the biomass from the biogas reactor is determined with the aid of the at least one measuring method,
- at least one selection criterion for selecting at least one enzyme-containing preparation from a given group of at least two enzyme-containing preparations is provided,
- if the determined feature value in the biomass deviates from the at least one reference value provided, the enzyme-containing preparation(s) selected according to the at least one selection criterion is/are added in such a quantity or over such a period of time to the biomass in the biogas reactor until the measured deviation disappears,
- in which the feature is the activity of at least one significant enzyme or one significant group of enzymes for the hydrolysis in the biomass and in which that enzyme or that enzyme group is added to the biomass in the biogas reactor, for which the determined activity is below the reference value provided.

2. The method according to Claim 1, wherein the reference value is determined from the empirical frequency distribution of the activity of the at least one enzyme in a random sample of biomass specimens from biogas reactors.

3. The method according to Claim 2, wherein the reference value is the 75% quantile of the empirical frequency distribution.

4. The method according to any one of Claims 1 to 3, wherein the reference value is empirically deduced from the effect of at least two enzyme-containing preparations on the decomposition of input substances or mixtures of input substances.

5. The method according to any one of Claims 1 to 3, wherein the reference value is empirically deduced from the material composition of the input substances or the biomass in a bioreactor.

6. The method according to any one of Claims 1 to 5, wherein, for the addition of the determined enzyme or groups of enzymes, that preparation or those preparations are selected from a group of at least two enzyme-containing preparations having different enzyme activities in a suitable combination such that the determined deviations of the enzyme activities from their reference values can be eliminated with the smallest total quantity or the lowest total costs of the enzyme-containing preparation(s) and the target value optimization method is used for this.

7. The method according to any one of Claims 1 to 6, in which the feature is the viscosity measured in the biomass from a biogas reactor and in which at least two preparations from a given group of at least two enzyme-containing preparations are added individually and/or in combination to the biomass from the biogas reactor, the temporal change of the viscosity with respect to a control is determined without the addition of an enzyme-containing preparation and that enzyme-containing preparation or that combination of enzyme-containing preparations from the given group of enzyme-containing preparations is added to the biomass in the biogas reactor, which, when empirically determined, produces the greatest reduction in the viscosity with respect to the control.

8. The method according to any one of Claims 1 to 7, in which the feature is the specific biogas formation from the biomass from a biogas reactor and in which at least two preparations from a given group of at least two enzyme-containing preparations are added individually and/or in combination to the biomass from the biogas reactor, the specific formation of biogas from the biomass is determined with respect to a control without the addition of an enzyme-containing preparation and that enzyme-containing preparation or that combination of enzyme-containing preparations from the given group of enzyme-containing preparations is added to the biomass in the biogas reactor, which produces the maximum specific biogas formation with respect to the control.

9. The method according to any one of Claims 1 to 8, according to which the reference value is determined from the empirical frequency distribution of the feature in a random sample of biomass specimens from biogas reactors.

10. The method according to Claim 9, wherein the reference value is a quantile of the empirical frequency distribution.

11. The method according to any one of Claims 1 to 10, according to which reference values for various groups of biogas plants are separately determined from a random sample and a group of biogas plants is defined in that these biogas plants have at least one of the following features in common: a common input substance, a common type of pre-treatment of the input substance, a common number of biogas reactors connected one behind the other, a common loading of the fermenter, common retention times in the biogas reactors, a common type of the agitators used, common operating temperatures in the biogas reactor, a common use of process-promoting auxiliary agents and/or common physical or biochemical parameters of the fermentation sludge, and it is determined in which group of biogas plants a biogas reactor falls and the reference values of the relevant group of biogas plants are enlisted for the method for generating biogas in the biogas reactor.

12. The method according to any one of Claims 7 to 11, in which the viscosity feature is preferably checked before the other features and any deviation is eliminated by an addition of one or more enzyme-containing preparations.

13. The method according to any one of Claims 1 to 12, in which the enzyme activities feature is preferably checked before the other features and any deviation is eliminated by an addition of one or more enzyme-containing preparations.

14. The method according to any one of Claims 1 to 13, in which at least one metabolite or a parameter is indicated with reference to the biomass from a biogas reactor, which serves as an indicator for a limiting, and at least one measuring method is indicated for measuring the concentration of the at least one metabolite or the value of the at least one parameter in the biomass, a reference value is provided for the concentration of the at least one metabolite or the at least one parameter in the biomass, and one or more enzyme-containing preparations is/are only added to the biomass in the specified biogas reactor as long as the concentration of the at least one metabolite measured in the biomass or the value of the at least one parameter is above or below the reference value.

## Revendications

1. Procédé de fabrication de biogaz dans un réacteur à biogaz, comportant les étapes suivantes :
- indication d'au moins une caractéristique relative à la biomasse sortant d'un réacteur à biogaz,
- indication d'au moins un procédé de mesure pour la mesure de l'au moins une caractéristique,
- mise à disposition d'au moins une valeur de référence pour l'au moins une caractéristique,
- fabrication de biogaz dans le réacteur à biogaz,
- détermination de la valeur de l'au moins une caractéristique dans la biomasse sortant du réacteur à biogaz à l'aide de l'au moins un procédé de mesure,
- mise à disposition d'au moins un critère de sélection pour la sélection d'au moins une préparation enzymatique à partir d'un groupe donné d'au moins deux préparations enzymatiques,
- si la valeur déterminée de la caractéristique dans la biomasse s'écarte de l'au moins une valeur de référence mise à disposition, la ou les préparations enzymatiques sélectionnées selon l'au moins un critère de sélection seront ajoutées à la biomasse dans le réacteur à biogaz dans une certaine quantité ou pendant une certaine durée jusqu'à disparition de l'écart mesuré,
- dans lequel la caractéristique consiste en l'activité d'au moins un enzyme pertinent pour l'hydrolyse ou d'un groupe d'enzymes pertinent dans la biomasse, et dans lequel l'enzyme ou le groupe d'enzymes ajouté à la biomasse dans le réacteur à biogaz est celui pour lequel l'activité déterminée se trouve en dessous de la valeur de référence mise à disposition.

2. Procédé selon la revendication 1, dans lequel la valeur de référence est déterminée à partir de la distribution de fréquence empirique de l'activité de l'au moins un enzyme dans un échantillon de prélèvements de biomasse issus de réacteurs à biogaz.

3. Procédé selon la revendication 2, dans lequel la valeur de référence est le quantile 75% de la distribution de fréquence empirique.

4. Procédé selon l'une des revendications 1 à 3, dans lequel la valeur de référence est dérivée empiriquement de l'effet d'au moins deux préparations enzymatiques sur la dégradation de matières entrantes ou de mélanges de matières entrantes.

5. Procédé selon l'une des revendications 1 à 3, dans lequel la valeur de référence est dérivée empiriquement de la composition matérielle des matières entrantes ou de la biomasse dans un réacteur à biogaz.

6. Procédé selon l'une des revendications 1 à 5, dans lequel, pour l'ajout de l'enzyme ou du groupe d'enzymes déterminé à partir d'un groupe d'au moins deux préparations enzymatiques avec différentes activités enzymatiques, la préparation ou les préparations sont choisies dans une combinaison appropriée de telle façon que les écarts déterminés des activités enzymatiques par rapport à leurs valeurs de référence peuvent être éliminés avec la quantité globale la plus petite ou les coûts globaux les plus faibles de la préparation enzymatique ou des préparations enzymatiques et que la méthode d'optimisation des valeurs cibles peut être utilisée à cette fin.

7. Procédé selon l'une des revendications 1 à 6, dans lequel la caractéristique consiste en la résistance mesurée dans la biomasse sortant d'un réacteur à biogaz et dans lequel au moins deux préparations issues d'un groupe donné d'au moins deux préparations enzymatiques sont ajoutés séparément et/ou de façon combinée à la biomasse sortant du réacteur à biogaz, la modification temporelle de la résistance est déterminée par rapport à un contrôle sans ajout de préparation enzymatique, et la préparation enzymatique ou la combinaison de préparations enzymatiques issues du groupe donné de préparations enzymatiques ajoutée à la biomasse dans le réacteur à biogaz est celle donnant la plus forte baisse de résistance par rapport au contrôle lors de la détermination empirique.

8. Procédé selon l'une des revendications 1 à 7, dans lequel la caractéristique consiste en la formation de biogaz spécifique à partir de la biomasse sortant d'un réacteur à biogaz et dans lequel, à partir d'un groupe donné d'au moins deux préparations enzymatiques, au moins deux préparations sont ajoutées séparément et/ou de façon combinée à la biomasse dans le réacteur à biogaz, la formation spécifique de biogaz à partir de la biomasse est déterminée par rapport à un contrôle sans ajout de préparation enzymatique, et la préparation enzymatique ou la combinaison de préparations enzymatiques issues du groupe donné de préparations enzymatiques ajoutée à la biomasse dans le réacteur à biogaz est celle donnant la formation de biogaz spécifique la plus élevée par rapport au contrôle.

9. Procédé selon l'une des revendications 1 à 8, selon lequel la valeur de référence est déterminée à partir de la distribution de fréquence empirique de la caractéristique dans un échantillon de prélèvements de biomasse issus de réacteurs à biogaz.

10. Procédé selon la revendication 9, dans lequel la valeur de référence est un quantile de la distribution de fréquence empirique.

11. Procédé selon l'une des revendications 1 à 10, selon lequel des valeurs de référence pour différents groupes d'installations de biogaz sont déterminées séparément à partir d'un échantillon, et un groupe d'installations de biogaz est défini par le fait que ces installations de biogaz ont en commun au moins l'une des caractéristiques suivantes : une même matière entrante, un même type de prétraitement de la matière entrante, un même nombre de réacteurs à biogaz montés en série, une même charge volumique du fermentateur, des mêmes temps de séjour dans les réacteurs à biogaz, un même type d'agitateurs utilisés, des mêmes températures de fonctionnement dans le réacteur à biogaz, un même emploi d'adjuvants favorisant le processus et/ou des mêmes paramètres physiques ou biochimiques de la boue de fermentation, et selon lequel il est déterminé de quel groupe d'installations de biogaz relève un réacteur à biogaz, et les valeurs de référence du groupe concerné d'installations de biogaz sont retenues pour le procédé de fabrication de biogaz dans le réacteur à biogaz.

12. Procédé selon l'une des revendications 7 à 11, dans lequel la caractéristique de résistance est vérifiée de préférence avant les autres caractéristiques et un écart éventuel est éliminé par l'ajout d'une ou de plusieurs préparations enzymatiques.

13. Procédé selon l'une des revendications 1 à 12, dans lequel la caractéristique d'activités enzymatiques est vérifiée de préférence avant les autres caractéristiques et un écart éventuel est éliminé par l'ajout d'une ou de plusieurs préparations enzymatiques.

14. Procédé selon l'une des revendications 1 à 13, dans lequel sont indiqués au moins un métabolite ou un paramètre relatif à la biomasse sortant d'un réacteur à biogaz, servant d'indicateur pour une limitation et au moins un procédé de mesure pour la mesure de la concentration de l'au moins un métabolite ou de la valeur de l'au moins un paramètre dans la biomasse, une valeur de référence pour la concentration de l'au moins un métabolite ou pour l'au moins un paramètre dans la biomasse est mise à disposition, et l'ajout d'une ou de plusieurs préparations enzymatiques à la biomasse dans le réacteur à biogaz déterminé n'est effectué que dans la mesure où la concentration de l'au moins un métabolite mesurée dans la biomasse ou la valeur de l'au moins un paramètre tombe en dessous ou au-dessus de la valeur de référence.
